(19) Europäisches Patentamt / European Patent Office / Office européen des brevets

(11) **EP 2 875 730 A1**

(12) **EUROPEAN PATENT APPLICATION**
published in accordance with Art. 153(4) EPC


(43) Date of publication:
**27.05.2015 Bulletin 2015/22**

(21) Application number: **13820583.6**

(22) Date of filing: **19.07.2013**

(51) Int Cl.:
***A01N 43/08*** *(2006.01)* ***A01G 7/06*** *(2006.01)*
***A01N 25/32*** *(2006.01)* ***A01N 43/16*** *(2006.01)*
***A01N 43/40*** *(2006.01)* ***A01N 43/54*** *(2006.01)*
***A01P 21/00*** *(2006.01)*

(86) International application number:
**PCT/JP2013/004430**

(87) International publication number:
**WO 2014/013744 (23.01.2014 Gazette 2014/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.07.2012 JP 2012161898**



(71) Applicants:
- **Nippon Soda Co., Ltd.**
  **Tokyo 100-8165 (JP)**
- **Shizuoka Prefecture**
  **Shizuoka-shi**
  **Shizuoka 420-8601 (JP)**

(72) Inventors:
- **KAGEYAMA, Chizuko**
  **Iwata-shi**
  **Shizuoka 438-0803 (JP)**
- **IYOZUMI, Hiroyuki**
  **Iwata-shi**
  **Shizuoka 438-0803 (JP)**
- **NUKUI, Hideki**
  **Iwata-shi**
  **Shizuoka 438-0803 (JP)**
- **KATO, Kimihiko**
  **Shizuoka-shi**
  **Shizuoka 420-8601 (JP)**
- **MANNEN, Junya**
  **Shizuoka-shi**
  **Shizuoka 420-8601 (JP)**
- **TOMIDA, Kazuyuki**
  **Odawara-shi**
  **Kanagawa 250-0280 (JP)**
- **SANO, Shinsuke**
  **Odawara-shi**
  **Kanagawa 250-0280 (JP)**
- **KATO, Hideki**
  **Odawara-shi**
  **Kanagawa 250-0280 (JP)**
- **MAKITA, Satoru**
  **Odawara-shi**
  **Kanagawa 250-0280 (JP)**
- **MIZUNO, Toshio**
  **Makinohara-shi**
  **Shizuoka 421-0412 (JP)**

(74) Representative: **Wills, Andrew Jonathan et al**
**Mewburn Ellis LLP**
**33 Gutter Lane**
**London EC2V 8AS (GB)**


(54) **METHOD FOR PROVIDING PLANTS WITH RESISTANCE TO STRESS**


(57) A method of providing a plant with stress resistance, comprising applying at least one substance (A) to the plant, said at least one substance (A) being selected from the group consisting of a compound represented by Formula (I) and the like and salts thereof. Phytotoxicity of a plant due to agricultural chemicals is reduced by providing the plant with stress resistance. [In Formula (I), $R^1$ to $R^4$ each independently represents a hydrogen atom, $-SO_3H$, $-PO_3H_2$, a glycosyl group or $-COR^{11}$. $R^{11}$ represents an unsubstituted or substituted C1 to C30 alkyl group or an unsubstituted or substituted C2 to C30 alkenyl group.].

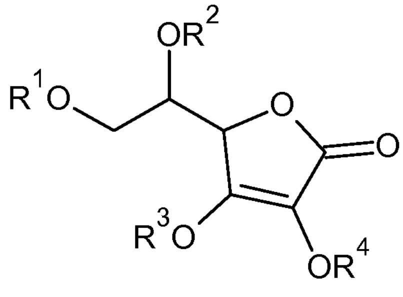
(I)


EP 2 875 730 A1

## Description

### TECHNICAL FIELD

The present invention relates to a method of providing a plant with stress resistance. More specifically, the present invention relates to a method of providing a plant with resistance to biological stress, physical stress or chemical stress which affects the growth of the plant.

### BACKGROUND ART

Plants grown at farmlands or ordinary home gardens are always exposed to various biological or non-biological stresses. In general, agricultural crops subjected to breed improvement tend to be less resistant to these stresses. In order to reduce biological stress such as agricultural pests and weeds to maintain a crop yield, agricultural chemicals are used such as fungicides, insecticides and herbicides. However, agricultural chemicals may have insufficient effects, and may cause phytotoxicity when improperly used, and may allow agricultural pests and weeds to develop resistance to the agricultural chemicals, and may pose concerns about safety for environmental life. Meanwhile, the right plant in the right place, breed improvement, irrigation, greenhouse, soil improvement and the like are utilized to respond environmental stress such as temperature, moisture, illuminance, soil pH and salt concentrations. Attempts have been made for conferring stress resistance using a plant growth regulator and the like, but effects have been unsatisfactory. Further, plant viral diseases may cause serious damage to key crops such as cereal crops, vegetables and fruit trees. However, to date, agricultural chemicals have not been found which sufficiently demonstrate practical effects against plant viral diseases.

Meanwhile, Non-patent Literature 1 describes that ascorbic acid is involved in disease resistance, hormone actions and the like, and Non-patent Literature 2 describes that ascorbic acid affects plant aging. However, even when ascorbic acid is externally given to a plant, its physiological effect is very limited because ascorbic acid is present at a high concentration in a plant body. Therefore, there will be almost no practical effect.

Nonetheless, Patent Literature 1 describes that a certain derivative of ascorbic acid demonstrates a preventive and curative effect against a plant virus disease, and proposes to apply it to a plant. Further, Patent Literature 2 discloses a composition comprising an antimicrobic antibiotic such as neomycin sulfate and ascorbic acid, and states that this composition can control a plant disease.

### CITATION LIST

#### Non-patent Literatures

Non-patent Literature 1: Vitamins 79 (2): 116-117 (2005)
Non-patent Literature 2: The Horticulture Journal, 6 (2): 169-175

#### Patent Literatures

Patent Literature 1: WO 2011/030816 A
Patent Literature 2: JP 2001-508808 A

### SUMMARY OF THE INVENTION

### PROBLEMS TO BE SOLVED BY THE INVENTION

An object of the present invention is to provide a method of providing a plant with resistance to biological stress, physical stress or chemical stress which affects the growth of the plant.

### MEANS FOR SOLVING THE PROBLEMS

As a result of conducting extensive studies to achieve the above object, the present inventors complete the present invention which has the following aspects.

[1] A method of providing a plant with stress resistance, wherein the method comprises applying at least one substance (A) to the plant, said at least one substance (A) being selected from the group consisting of compounds represented by Formula (I), compounds represented by Formula (II) and salts thereof.

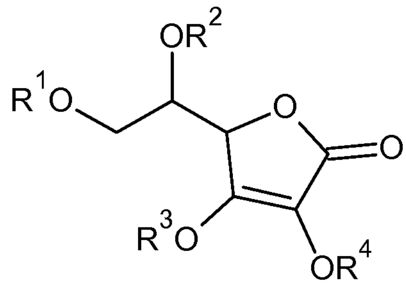
(I)

[In Formula (I), $R^1$ to $R^4$ each independently represents a hydrogen atom, $-SO_3H$, $-PO_3H_2$, a glycosyl group, or $-COR^{11}$. $R^{11}$ represents an unsubstituted or substituted C1 to C30 alkyl group or an unsubstituted or substituted C2 to C30 alkenyl group.]

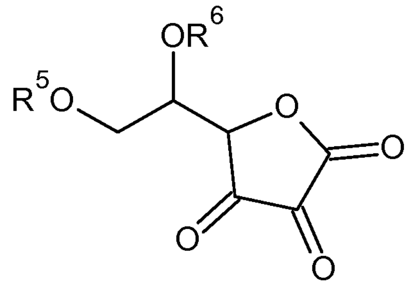
(II)

[In Formula (II), $R^5$ and $R^6$ each independently represents a hydrogen atom, $-SO_3H$, $-PO_3H_2$, a glycosyl group or $-COR^{11}$. $R^{11}$ represents an unsubstituted or substituted C1 to C30 alkyl group or an unsubstituted or substituted C2 to C30 alkenyl group.]

[2] The method according to [1], wherein the substance (A) is a compound represented by Formula (I) [provided that $R^1$ to $R^4$ are not each simultaneously a hydrogen atom] or a salt thereof.

[3] The method according to [1], wherein the substance (A) is a compound represented by Formula (I) [provided that at least one of $R^1$ to $R^4$ represents $-COR^{11}$, and $R^{11}$ represents an unsubstituted or substituted C12 to C30 alkyl group or an unsubstituted or substituted C12 to C30 alkenyl group.] or a salt thereof.

[4] The method according to [1], wherein the substance (A) is a compound represented by Formula (I) [provided that $R^1$ to $R^4$ each independently represents a hydrogen atom or $-COR^{11}$, and at least one of $R^1$ to $R^4$ represents $-COR^{11}$. $R^{11}$ represents an unsubstituted or substituted C1 to C30 alkyl group or an unsubstituted or substituted C2 to C30 alkenyl group. $R^{11}$ in at least one of $-COR^{11}$ represents an unsubstituted or substituted C12 to C30 alkyl group or an unsubstituted or substituted C12 to C30 alkenyl group.] or a salt thereof.

[5] The method according to [1], wherein the substance (A) is a composition comprising a water soluble substance (A1) of those selected from the group consisting of compounds represented by Formula (I), compounds represented by Formula (II) and salts thereof; and a lipid soluble substance (A2) of those selected from the group consisting of compounds represented by Formula (I), compounds represented by Formula (II) and salts thereof.

[6] The method according to any one of [1] to [5], wherein the stress is biological stress due to plant viruses, phytopathogenic bacteria, phytopathogenic filamentous fungi, pests or weeds; or physical or chemical stress due to high temperature, low temperature, high illuminance, low illuminance, excessive humidity, dryness, salt, acidity, agricultural chemicals, chemical substances or heavy metals.

[7] A stress resistance conferring composition for a plant, wherein the composition comprises at least two substances (A) selected from the group consisting of compounds represented by Formula (I), compounds represented by Formula (II) and salts thereof.

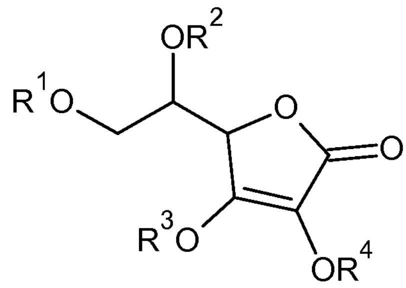

(I)

[In Formula (I), $R^1$ to $R^4$ each independently represents a hydrogen atom, $-SO_3H$, $-PO_3H_2$, a glycosyl group or $-COR^{11}$. $R^{11}$ represents an unsubstituted or substituted C1 to C30 alkyl group or an unsubstituted or substituted C2 to C30 alkenyl group.]

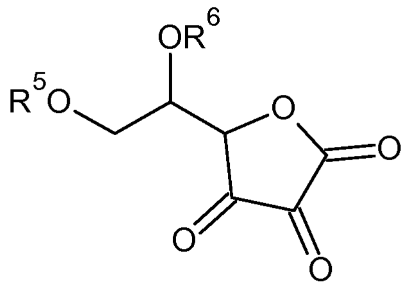

(II)

[In Formula (II), $R^5$ and $R^6$ each independently represents a hydrogen atom, $-SO_3H$, $-PO_3H_2$, a glycosyl group or $-COR^{11}$. $R^{11}$ represents an unsubstituted or substituted C1 to C30 alkyl group or an unsubstituted or substituted C2 to C30 alkenyl group.]

[8] The composition according to [7], wherein one of the substances (A) is a water soluble substance (A1) of those selected from the group consisting of compounds represented by Formula (I), compounds represented by Formula (II) and salts thereof; and another of the substances (A) is a lipid soluble substance (A2) of those selected from the group consisting of compounds represented by Formula (I), compounds represented by Formula (II) and salts thereof.

[9] A stress resistance conferring composition for a plant, wherein the composition comprises at least one water soluble substance (A1) selected from the group consisting of compounds represented by Formula (Ia), compounds represented by Formula (IIa) and salts thereof; and

at least one lipid soluble substance (A2) selected from the group consisting of compounds represented by Formula (Ib), compounds represented by Formula (IIb) and salts thereof.

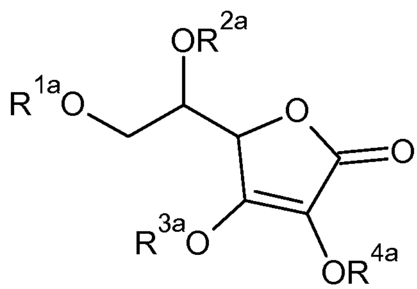

(Ia)

[In Formula (Ia), $R^{1a}$ to $R^{4a}$ each independently represents a hydrogen atom, $-SO_3H$, $-PO_3H_2$ or a glycosyl group.]

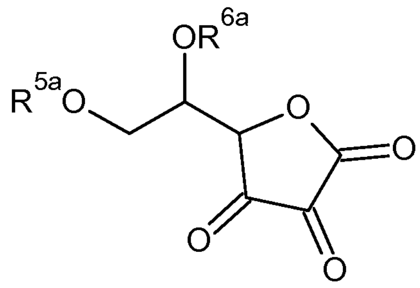

(IIa)

[In Formula (IIa), $R^{5a}$ to $R^{6a}$ each independently represents a hydrogen atom, $-SO_3H$, $-PO_3H_2$ or a glycosyl group.]

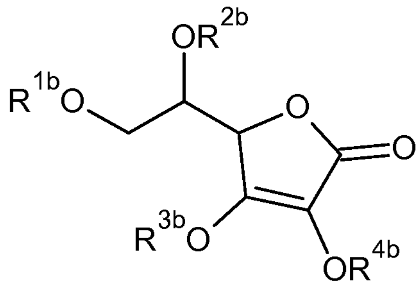

(Ib)

[In Formula (Ib), $R^{1b}$ to $R^{4b}$ each independently represents a hydrogen atom or $-COR^{11}$. At least one of $R^{1b}$ to $R^{4b}$ represents $-COR^{11}$, and $R^{11}$ represents an unsubstituted or substituted C1 to C30 alkyl group or an unsubstituted or substituted C2 to C30 alkenyl group.]

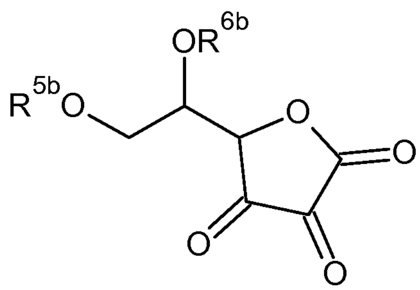

(IIb)

[In Formula (IIb), $R^{5b}$ and $R^{6b}$ each independently represents a hydrogen atom or $-COR^{11}$. At least one of $R^{5b}$ and $R^{6b}$ represents $-COR^{11}$, and $R^{11}$ represents an unsubstituted or substituted C1 to C30 alkyl group or an unsubstituted or substituted C2 to C30 alkenyl group.]

[10] A method of reducing phytotoxicity of a plant due to an agricultural chemical, wherein the method comprises providing the plant with stress resistance by the method according to any one of [1] to [6].

[11] The method of reducing phytotoxicity of a plant due to an agricultural chemical according to claim 10, wherein the agricultural chemical comprises at least one selected from the group consisting of fungicides, insecticides, plant growth regulators and herbicides.

ADVANTAGEOUS EFFECTS OF THE INVENTION

The method according to the present invention can provide a plant with resistance to biological stress, physical stress or chemical stress which affects the growth of the plant. As a result, for example, phytotoxicity due to agricultural chemicals comprising a substance and the like which may affect a physiological function of a plant can be reduced, and damage due to plant diseases including virus diseases can be reduced. Moreover, even under poor environmental conditions such as high temperature, low temperature, dryness and soil conditions, reduced crop yield, deteriorated quality and the like can be prevented.

EMBODIMENTS FOR CARRYING OUT THE INVENTION

The method of providing a plant with stress resistance according to the present invention comprises applying the substance (A) to a plant.

(Substance (A))

The substance (A) is at least one selected from the group consisting of compounds represented by Formula (I), compounds represented by Formula (II) and salts thereof.
In Formula (I), $R^1$ to $R^4$ each independently represents a hydrogen atom, $-SO_3H$, $-PO_3H_2$, a glycosyl group or $-COR^{11}$.
In Formula (II), $R^5$ and $R^6$ each independently represents a hydrogen atom, $-SO_3H$, $-PO_3H_2$, a glycosyl group or $-COR^{11}$.
The glycosyl group is a sugar residue such as a monosaccharide or a low molecular weight oligosaccharide (which is, specifically, a partial structure of a molecule in which a hemiacetal hydroxy group at a sugar portion is removed to give a connecting position). Examples of monosaccharides include glucose, galactose, fructose, rhamnose and the like, and examples of oligosaccharides include rutinose, vicianose, lactose, maltose, sucrose and the like. Therefore, examples of glycosyl groups include a glucosyl group, a galactosyl group, a fructosyl group, a rhamnosyl group and the like. Further, glycosyl groups include disaccharide groups in which any combination of these groups are connected in the $1\rightarrow2$ linkage, the $1\rightarrow3$ linkage, the $1\rightarrow4$ linkage or the $1\rightarrow6$ linkage.
$R^{11}$ in $-COR^{11}$ represents an unsubstituted or substituted C1 to C30 alkyl group or an unsubstituted or substituted C2 to C30 alkenyl group.
As used herein, the term "unsubstituted" means that a corresponding group comprises only a group serving as a mother nucleus. Note that when described only under the name of a group serving as a mother nucleus without a description of "substituted", it means "unsubstituted" unless otherwise stated.
Meanwhile, the term "substituted" means that any hydrogen atom in a group serving as a mother nucleus is substituted with a group having a structure which is different from or the same as the mother nucleus. Therefore, the term "substituent" is another group substituted on a group serving as a mother nucleus. The number of substituents may be 1, or may be 2 or more. Two or more substituents may be the same, or may be different. For example, a substituted C1 to C30 alkyl group is a group having a structure in which the group serving as a mother nucleus is a C1 to C30 alkyl group, and any hydrogen atom thereof is substituted with a group having a different structure ("substituent").
A "C1 to C30 alkyl group" in $R^{11}$ is a saturated hydrocarbon group comprising 1 to 30 carbon atoms. A C1 to C30 alkyl group may be a linear chain, or may be a branched chain. Examples of C1 to C30 alkyl groups include a methyl group, an ethyl group, an n-propyl group, an n-butyl group, an n-pentyl group, an n-hexyl group, an n-heptyl group, an n-octyl group, an i-propyl group, an i-butyl group, an s-butyl group, a t-butyl group, an i-pentyl group, a neopentyl group, a 2-methylbutyl group, a 2,2-dimethylpropyl group, an i-hexyl group, a heptyl group, an octyl group, a nonyl group, a decyl group, an undecyl group, a dodecyl group, a tridecyl group, a tetradecyl group (a myristyl group), a pentadecyl group, a hexadecyl group (a cetyl group, a palmityl group), a heptadecyl group, an octadecyl group (a stearyl group), a nonadecyl group, an icosyl group, a henicosyl group, a triacontyl group and the like.
A "C2 to C30 alkenyl group" in $R^{11}$ is an unsaturated hydrocarbon group comprising 2 to 30 carbon atoms having at least one carbon-carbon double bond. A C2 to C30 alkenyl group may be a linear chain, or may be a branched chain. Examples of C2 to C30 alkenyl groups include a vinyl group, a 1-propenyl group, an isopropenyl group, an allyl group, a 1-butenyl group, a 2-butenyl group, a 3-butenyl group, a 1-pentenyl group, a 2-pentenyl group, a 3-pentenyl group, a 4-pentenyl group, a 1-hexenyl group, a 2-hexenyl group, a 3-hexenyl group, a 4-hexenyl group, a 5-hexenyl group, a 1-heptenyl group, a 6-heptenyl group, a 1-octenyl group, a 7-octenyl group, a 1-methyl-allyl group, a 2-methyl-allyl group, a 1-methyl-2-butenyl group, a 2-methyl-2-butenyl group, an octenyl group, a nonenyl group, a decenyl group, an undecenyl group, a dodecenyl group, a tridecenyl group, a tetradecenyl group, a pentadecenyl group, a hexadecenyl group, a heptadecenyl group, an octadecenyl group, a nonadecenyl group, an icosenyl group, a henicosenyl group, a triacontenyl group and the like.
Examples of groups which can be a "substituent" in the C1 to C30 alkyl group or the C2 to C30 alkenyl group include a hydroxyl group; a mercapto group; an amino group; a nitro group; a halogen atom such as a chlorine atom, a fluorine atom, a bromine atom; an alkoxy group such as a methoxy group, an ethoxy group, an isopropoxy group, an n-propoxy group, an n-butoxy group, an isobutoxy group, an s-butoxy group, a t-butoxy group; an aryloxy group such as a phenoxy group, a 1-naphthyloxy group; a haloalkoxy group such as a fluoromethoxy group, a difluoromethoxy group, a trifluoromethoxy group, a 2-chloroethoxy group, a 2,2,2-trichloroethoxy group, a 1,1,1,3,3,3-hexafluoro-2-propoxy group; an alkylthio group such as a methylthio group, an ethylthio group; an arylthio group such as a phenylthio group, a 1-naphthylthio group; an alkylamino group such as a methylamino group, a diethylamino group; an arylamino group such as an anilino group, a 1-naphthyl amino group; a cyano group and the like.

Preferably, the above $R^{11}$ represents an unsubstituted or substituted C8 to C20 alkyl group or an unsubstituted or substituted C8 to C20 alkenyl group.

The substance (A) is preferably a compound represented by Formula (I) or a salt thereof. Further, preferably, $R^1$ to $R^4$ in Formula (I) are not simultaneously hydrogen atoms.

Moreover, the substance (A) is preferably a compound represented by Formula (I) [at least one of $R^1$ to $R^4$ represents $-COR^{11}$. $R^{11}$ represents an unsubstituted or substituted C12 to C30 alkyl group or an unsubstituted or substituted C12 to C30 alkenyl group.] or a salt thereof.

Examples of "C12 to C30 alkyl groups" include a dodecyl group, a tridecyl group, a tetradecyl group (a myristyl group), a pentadecyl group, a hexadecyl group (a cetyl group, a palmityl group), a heptadecyl group, an octadecyl group (a stearyl group), a nonadecyl group, an icosyl group, a henicosyl group, a triacontyl group and the like.

Examples of "Substituted C12 to C30 alkyl groups" include a 2-hydroxytridecyl group, a 1-hydroxypentadecyl group, an 11-hydroxyheptadecyl group, a 1-aminoheptadecyl group and the like.

Examples of "C12 to C30 alkenyl groups" include a dodecenyl group, a tridecenyl group, a tetradecenyl group, a pentadecenyl group, a hexadecenyl group, a heptadecenyl group, an octadecenyl group, a nonadecenyl group, an icosenyl group, a henicosenyl group, a triacontenyl group and the like.

Examples of "substituted C12 to C30 alkenyl groups" include a 7-hydroxy-8-pentadecenyl group, a 1-hydroxy-8-heptadecenyl group, a 1-amino-8-heptadecenyl group and the like.

Further, the substance (A) is preferably a compound represented by Formula (I) [$R^1$ to $R^4$ each independently represents a hydrogen atom or $-COR^{11}$, and at least one of $R^1$ to $R^4$ represents $-COR^{11}$, and $R^{11}$ represents an unsubstituted or substituted C1 to C30 alkyl group or an unsubstituted or substituted C2 to C30 alkenyl group, and $R^{11}$ in at least one of $-COR^{11}$ represents an unsubstituted or substituted C12 to C30 alkyl group or an unsubstituted or substituted C12 to C30 alkenyl group.] or a salt thereof.

Specific examples of the substance (A) as described above can include ascorbic acid 6-myristate, ascorbic acid 6-palmitate, ascorbic acid 6-stearate, ascorbic acid 2-myristate, ascorbic acid 2-palmitate, ascorbic acid 2-stearate, ascorbic acid 2,6-dimyristate, ascorbic acid 2,6-dipalmitate, ascorbic acid 2,6-distearate and the like.

There is no particular limitation for salts of a compound represented by Formula (I) and salts of a compound represented by Formula (II) as long as they are agriculturally and horticulturally acceptable salts. They can include, for example, an alkali metal salt such as a sodium salt, a potassium salt; an alkaline earth metal salt such as a calcium salt, a magnesium salt and the like.

The substance (A) used for the present invention can be obtained by a known synthesis approach. For example, an esterification reaction of a fatty acid compound with ascorbic acid for introducing $-COR^{11}$ into any of $R^1$ to $R^4$, an esterification reaction of a phosphoric acid compound with ascorbic acid for introducing $-PO_3H_2$ into any of $R^1$ to $R^4$, an esterification reaction of a sulfuric acid compound with ascorbic acid for introducing $-SO_3H$ into any of $R^1$ to $R^4$, and other known reactions can be used for synthesis. Further, the substances (A) obtained by the aforementioned synthesis methods can be purified by a known method such as extraction, distillation, chromatography. Moreover, many of the substances (A) used for the present invention are commercially available, and therefore it is also possible to use them.

The structure of the substance (A) can be identified or confirmed by a known analytical means such as an IR spectrum, an NMR spectrum, a mass spectrum, elementary analysis.

The substance (A) may be used alone, but is preferably used in combination of at least two. In a case where a combination of two is used, the substance (A) is preferably a composition comprising a water soluble substance (A1) of those selected from the group consisting of compounds represented by Formula (I), compounds represented by Formula (II) and salts thereof; and a lipid soluble substance (A2) of those selected from the group consisting of compounds represented by Formula (I), compounds represented by Formula (II) and salts thereof because an effect of the substance (A) is synergistically enhanced.

In a case where a combination of two is used, more specifically, the substance (A) is preferably a composition comprising at least one water soluble substance (A1) selected from the group consisting of compounds represented by Formula (Ia), compounds represented by Formula (IIa) and salts thereof; and at least one lipid soluble substance (A2) selected from the group consisting of compounds represented by Formula (Ib), compounds represented by Formula (IIb) and salts thereof.

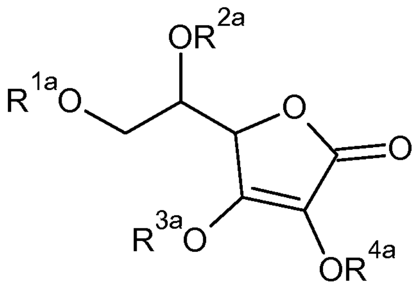

(Ia)

[In Formula (Ia), $R^{1a}$ to $R^{4a}$ each independently represents a hydrogen atom, $-SO_3H$, $-PO_3H_2$ or a glycosyl group.]

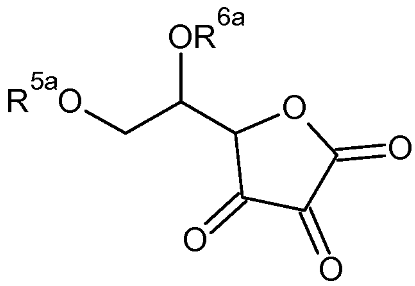

(IIa)

[In Formula (IIa), $R^{5a}$ to $R^{6a}$ each independently represents a hydrogen atom, $-SO_3H$, $-PO_3H_2$ or a glycosyl group.]

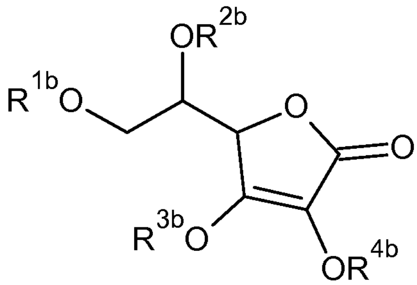

(Ib)

[In Formula (Ib), $R^{1b}$ to $R^{4b}$ each independently represents a hydrogen atom or $-COR^{11}$. At least one of $R^{1b}$ to $R^{4b}$ represents $-COR^{11}$, and $R^{11}$ represents an unsubstituted or substituted C1 to C30 alkyl group or an unsubstituted or substituted C2 to C30 alkenyl group, preferably an unsubstituted or substituted C12 to C30 alkyl group or an unsubstituted or substituted C12 to C30 alkenyl group.]

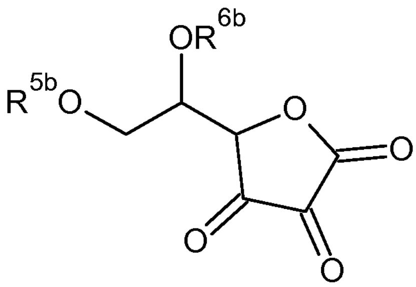

(IIb)

[In Formula (IIb), $R^{5b}$ and $R^{6b}$ each independently represents a hydrogen atom or $-COR^{11}$. At least one of $R^{5b}$ and $R^{6b}$ represents $-COR^{11}$, and $R^{11}$ represents an unsubstituted or substituted C1 to C30 alkyl group or an unsubstituted or substituted C2 to C30 alkenyl group, preferably an unsubstituted or substituted C12 to C30 alkyl group or an unsubstituted or substituted C12 to C30 alkenyl group.]

The mass ratio of the lipid soluble substance (A2) to the water soluble substance (A1) is usually from 0.001 to

1000, preferably from 0.1 to 10.

The substance (A) can be prepared into a formulation such as a wettable powder, an emulsifiable concentrate, a water soluble powder, a water dispersible granule, a dust, a tablet and the like. There is no particular limitation for a method of preparing a formulation, and a known preparation method can be used depending on a dosage form.

There is no particular limitation for a method of applying the substance (A) to a plant, and a known application method in the field of agriculture and horticulture can be used. Further, an application method to a plant can be suitably determined depending on the type and the like of a target plant. For example, preferred modes of application can include foliage application, dipping treatment, soil irrigation, seed treatment, water culture medium treatment, smoking treatment, ordinary temperature fogging treatment and the like. The method according to the present invention may be used without limitation by cultivation forms such as soil cultivation and hydroponic cultivation. Further, excellent effects can be achieved even when used in a special environment such as meristem culture. An application amount of the substance (A) according to the present invention can be suitably determined depending on meteorological conditions, formulation forms, application times, application methods, application places, target diseases to be controlled, target crops and the like.

There is no particular limitation for plants to which the method according to the present invention can be employed, and they may be either edible plants or non-edible plants. Examples of the target plants include cereal crops such as rice plant, wheat, corn; legumes such as soybean, azuki bean, peanut; fruit trees such as citrus, apple, pear, grape, peach; vegetables such as tomato, lettuce, cabbage, onion, green onion, bell pepper; pepos such as cucumber, watermelon, melon, pumpkin; root vegetables such as potato, sweet potato, Chinese yam, carrot, radish; crops for processing such as cotton, sugarbeet, hop, sugarcane, rubber tree, coffee, tobacco, tea; grass such as ryegrass, timothy, orchard grass; lawn grasses such as bentgrass, Zoysia grass, and the like.

The method according to the present invention can provide a plant with stress resistance. Such stresses include biological stress due to plant viruses, phytopathogenic bacteria, phytopathogenic filamentous fungi, agricultural pests, weeds, or microorganisms used as biological agricultural chemicals or arthropods; physical or chemical stress due to high temperature, low temperature, high illuminance, low illuminance, excessive humidity, dryness, salinity, acidity, agricultural chemicals, chemical substances or heavy metals.

There is no particular limitation for plant viruses which may cause stress. For example, preferably, they can include gemini viruses having a single stranded DNA as the genome, cauliflower mosaic virus having double stranded DNA as the genome, tobacco mosaic virus, tomato bushy stunt virus having a single stranded RNA as the genome, rice ragged stunt virus having double stranded RNA as the genome and the like.

There is no particular limitation for phytopathogenic bacteria which may cause stress. For example, they include Burkholderia plantarii, Acidovorax avenae, Burkholderia glumae, Xanthomonas campestris pv.oryzae, Pseudomonas lachrymans, Erwinia carotovora and the like.

There is no particular limitation for phytopathogenic filamentous fungi which may cause stress. For example, they include Pyricularia oryzae, Gibberella fujikuroi, Cochliobolus miyabeanus, Erysiphe graminis f.sp.tritici, Gibberella zeae, Puccinia recondita, Septoria tritici, Leptosphaeria nodorum, Ustilago tritici, Sphaerotheca fuliginea, Pseudoperonospora cubensis, Mycosphaerella melonis, Fusarium oxysporum, Botrytis cinerea, Colletotrichum orbiculare, Cladosporium cucumerinum, Corynespora cassicola, Cladosporium fulvum, Phytophthora infestans and the like.

There is no particular limitation for pests which may cause stress, and examples of the pests include:

Lepidoptera pests, for example, Spodoptera frugiperda, Leucania, Spodoptera litura, Agrotis ipsilon, Adoxophyes honmai, Homona magnanima, Carposina niponensis Walsingham, Cydia molesta, Phyllocnistis citrella, Caloptilia theivora, Phyllonorycter ringoniella, Lymantria dispar, Euproctis pseudoconspersa, Chilo suppressalis, Cnaphalocrocis medinalis, Ostrinia nubilalis, Hyphantria cunea, Cadra cautella, the genus Heliothis, the genus Helicoverpa, the genus Agrotis, Tinea translucens, Ostrinia furnacalis, Pieris brassicae, Heliothis virescens, Plutella xylostella, cutworm (a kind of Noctuidae) and the like;

Hemiptera pests, for example, Aphidae such as Lipaphis erysimi, Rhopalosiphum padi, Myzus persicaem, Aphis gossypii, Aphis favae; Aleyrodidae such as Trialeurodes vaporariorum, Bemisia tabaci, Bemisia argentifolii; Pyrrhocoroidea, Riptortus clavatus, Nezara antennata, Unaspis yanonensis, Pseudococcus longispinis, Psylla pyricola, Stephanitis nashi, Nilaparvata lugens, Laodelphax straitellus, Sogatella furcifera, Nephotettix cincticeps and the like;

Coleoptera pests, for example, Phyllotreta striolata, Aulacophora femoralis, Leptinotarsa decemlineata, Phaedon cochleariae, Lissorhoptrus oryzophilus, Sitophilus zeamais, Callosobruchus chinensis, Popillia japonica, Anomala rufocuprea, corn rootwarm, the genus Diabrotic, Lasioderma serricorne, Lyctus brunneus, Monochamus alternatus, Anoplophora malasiaca, the genus Agriote, Epilachna vigintioctopunctata, Trogossitidae, Anthonomus grandis and the like;

Orthoptera pests, for example, locust, Locusta migratoria and the like;

Thysanoptera pests, for example, Thrips palmi, Scirtothrips dorsalis, Thrips tabaci, Frankliniella intonsa and the like;

Diptera pests, for example, Dacus cucurbitae, Bactrocera dorsalis, Agromyza oryzae and the like;

Mites, for example, Tetranychidae such as Tetranychus urticae, Tetranychus cinnabarinus, Tetranychus kanzawa,

Panonychus citri, Panonychus ulmi, Tenuipalpidae; Aculops pelekassi, Aculus schlechtendali, Polyphagotarsonemus latus, Rhizoglyphus robini and the like.

Among these, agricultural pests for which application are particularly preferred include Aphidoidea, Aleyrodoidea, Thripidae, and Tetranychidae.

There is no particular limitation for weeds which may cause stress, and examples of them include gramineous weeds such as Echinochloa crus-galli, Sorghum bicolor, Setaria faberi Herrm, Setaria viridis, Setaria glauca, Alopecurus aequalis, Digitaria ciliaris, Eleusine indica, Poa annua; Compositae weeds such as Xanthium strumarium, Ambrosia artemisiifolia, Ambrosia trifida, Erigeron annuus, Erigeron philadelphicus, Erigeron canadensis, Conyza sumatrensis, Youngia japonica, Conyza bonariensis, Gnaphalium japonicum, Bidens, Artemisia princeps; Oxalis corniculata, Plantago asiatica, Polygonaceae, Capsella bursa-pastoris, Cardamine flexuosa, Galium aparine Abutilon theophrasti, Hydrocotyle sibthorpioides, Solanum nigrum, Ipomoea hederacea, Amaranthus lividus, Amaranthus viridis, Amaranthus retroflexus, Chenopodium album var. centrorubrum, Chenopodium album, Viola verecunda, Sida spinosa, Trifolium repens, Senna obtusifolia, Scirpus hotarui, Eleocharis acicularis, Cyperus serotinus Rottb, Monochoria vaginalis, Lindernia procumbens, Elatine triandra, Sagittaria pygmaea and the like. Preferably, they include plant parasites such as the genus Striga of Scrophulariaceae and the genus Orobanche of Orobanchaceae, which are parasitic on cereal crops, legumes, eggplant, tomato and the like in Africa, causing significant decrease in crop yields. Further, they include Amaranthus palmeri of Amaranthaceae, Ambrosia artemisiifolia and Erigeron canadensis of Asteraceae, which are glyphosate resistant weeds.

There is no particular limitation for high temperature and low temperature which may cause stress. They include, for example, high temperature injury and low temperature injury which may decrease the growth and quality of rice plant, high temperature injury which may decrease the fruit setting percentage of Solanaceae crops such as tomato, high temperature injury which tends to occur particularly in tunnel cultivation and greenhouse cultivation of lettuce and the like, high temperature injury which may inhibit the growth of turves, freezing and frost damage to tea plant and fruit trees such as citrus and the like.

There is no particular limitation for excessive humidity and dryness which may cause stress. For example, they are the poor growth of crops due to excessive humidity resulting from excessive rain fall, irrigation and poorly drained soil; or the decrease in disease resistance; or the wilt of crops due to dryness resulting from the shortage of rain fall and irrigation, sandy soil and the like.

There is no particular limitation for physical properties of soil which may cause stress. For example, they are growth disorders of crops in salty soil, acidic soil or alkaline soil and the like. Among these, effects on the poor growth in salty soil and acidic soil, in particular, effects on the poor growth of crops which are weak to acidic soil such as spinach, garden pea, fava bean, onion, asparagus, lettuce, burdock are significant, and it is effective for improving the yields and qualities of these crops.

There is no particular limitation for chemical substances which may cause stress, including at least one compound selected from agricultural chemicals such as herbicides, growth regulators, plant hormones, disease resistance inducers, fungicides, insecticides, miticides; fertilizers; surfactants; allelopathy substances produced by other plants which affects crops and the like.

There is no particular limitation for agricultural chemicals which may cause stress, and examples of the chemicals include those described as substances which may affect a physiological function of a plant.

Phytotoxicity which may cause stress is, for example, phytotoxicity when treated at a concentration above the usage standard and when applied to non-intended crops, and in addition, phytotoxicity occurring under high temperature and strong light conditions and the like. Further, the application range of agricultural chemicals can be extended wider than the conventional application range because the present invention controls those phytotoxicitys.

There is no particular limitation for heavy metals which may cause stress, and examples of the heavy metals include iron, zinc, copper, manganese, nickel, cobalt, tin, chromium, lead, cadmium, mercury, arsenic and the like.

Phytotoxicity of a plant due to agricultural chemicals can be reduced by the method according to the present invention. Examples of agricultural chemicals include herbicides; growth regulators; plant hormones; resistance inducers against pathogens; fungicides, insecticides, miticides, repellents, fertilizers, surfactants which may cause phytotoxicity when used at a high concentration; and the like. Among these, preferred is at least one selected from the group consisting of fungicides, insecticides, plant growth regulators and herbicides. Further, the agricultural chemical is preferably a respiratory inhibitor.

Furthermore, the agricultural chemical is preferably a strobilurin compound.

Examples of fungicides include those such as captan, folpet, thiuram, dilam, zineb, maneb, mancozeb, propineb, polycarbamate, chlorothalonil, quintozene, captaphore, iprodione, procymidone, fluoroimide, mepronil, flutolanil, pencycuron, oxycarboxin, fosetylaluminium, propamocarb, hexaconazole, imibenconazole, tebuconazole, difenoconazole, prothioconazole, fenbuconazole, diclobutrazol, bitertanol, myclobutanil, flusilazole, hexaconazole, etaconazole, fluotrimazole, triadimefon, triadimenol, flutriafen, penconazole, diniconazole, cyproconazole, fenarimol, triflumizole, prochloraz, imazalil, kresoxim-methyl, trifloxystrobin, azoxystrobin, pyraclostrobin, orysastrobin, pefurazoate, tridemorph, fenpropimorph, trifolin, buthiobate, pyrifenox, anilazine, polyoxin, metalaxyl, oxadixyl, furalaxyl, isoprothiolane, probenazole, pyrrolnitrin, blasticidin S, kasugamycin, validamycin, dihydrostreptomycin sulfate, benomyl, carbendazim, thiophanate-methyl, hymexazol, basic copper chloride, basic copper sulfate, fentinacetate, triphenyltin hydroxide, diethofencarb, chinomethionate, binapacryl, lecithin, sodium bicarbonate, dithianon, dinocap, fenaminosulf, dichlomedin, guazatine, dodine, IBP, edifenphos, mepanipyrim, ferimzone, trichlamid, metasulfocarb, fluazinam, etoquinolak, dimethomorph, pyroquilon, tecloftalam, fthalide, phenazine oxide, thiabendazole, tricyclazole, vincrozoline, cymoxanil, guazatine, propamocarb hydrochloride, oxolinic acid, cyflufenamid, iminoctadine, triazine, fenhexamid, cyazofamid, cyprodinil, carpropamide, boscalid. They also include resistance inducers against a pathogen such as probenazole, tiadinil.

Among these, particularly preferred are strobilurin based fungicides such as kresoxim-methyl, trifloxystrobin, azoxystrobin, pyraclostrobin, orysastrobin.

Herbicides include 2,4-D, MCPA, clomeprop, dicamba, chlorotoluron, diuron, linuron, isouron, fenuron, neburon, simazine, atrazine, simetryn, prometryn, hexazinone, propazine, desmetryn, terbumeton, propanil, bromoxynil, ioxynil, pyridate, chloridazon, bentazone, chlomethoxyfen, bifenox, acifluorfen sodium salt, flumioxazin, thidiazimin, oxadiazon, sulfentrazone, pentoxazone, pyraclonil, pyrazolynate, pyrazoxyfen, benzofenap, mesotrione, isoxaflutole, isoxachlortole, amitrole, aclonifen, diflufenican, benzobicyclon, diclofop-methyl, fluazifop-butyl, alloxydim sodium salt, clethodim, sethoxydim, tralkoxydim, tepraloxydim, bensulfuron-methyl, pyrazosulfuron-ethyl, rimsulfuron, imazosulfuron, prosulfuron, flumetsulam, diclosulam, metosulam, imazapyr, imazaquin, pyrithiobac-sodium salt, bispyribac-sodium salt, pyriminobac-methyl, flucabazone, propoxycarbazone, glyphosate, glyphosate ammonium salt, glufosinate, trifluralin, pendimethalin, benfluralin, prodiamine, propham, dithiopyr, alachlor, metolachlor, pethoxamid, acetochlor, propachlor, dimethenamid, diphenamid, napropamide, mefenacet, fentrazamide, molinate, dimepiperate, cycloate, esprocarb, thiobencarb, thiocarbazil, bensulide, dalapon, asulam, DNOC, dinoseb, flupoxam, traiziflam, quinchlorac, cinmethylin, dazomet, dymron, etobenzanide, oxaziclomefone, pyributicarband the like.

Examples of insecticides include organophosphate based and carbamate based insecticides such as fenthion, fenitrothion, diazinon, chlorpyrifos, ESP, vamidothion, phenthoate, dimethoate, formothion, malathion, trichlorfon, thiometon, phosmet, dichlorvos, acephate, EPBP, methylparathion, oxydemeton-methyl, ethion, salithion, cyanophos, isoxathion, pyridaphenthion, phosalone, methidathion, sulprofos, chlorfenvinphos, tetrachlorvinphos, dimethylvinphos, propaphos, isofenphos, ethylthiometon, prophenophos, pyraclophos, monocrotophos, azinephosmethyl, aldicarb, methomyl, thiodicarb, carbofuran, carbosulfane, benfuracarb, furathiocarb, propoxur, BPMC, MTMC, MIPC, carbaryl, pirimicarb, ethiofencarb, phenoxycarb, cartap, thiocyclam, bensultap; pyrethroid based insecticides such as permethrin, cypermethrin, deltamethrin, fenvalerate, fenpropathrin, pyrethrin, allethrin, tetramethrin, resmethrin, dimethrin, propathrin, phenothrin, prothrin, fluvalinate, cyfluthrin, cyhalothrin, flucythrinate, etofenprox, cycloprothrin, tralomethrin, silafluofen, acrinathrin; neonicotinoid based insecticides such as imidacloprid, acetamiprid, nitenpyram, thiacloprid, clothianidin, thiamethoxam, dinotefuran, nithiazine; benzoylphenylurea based insecticides such as diflubenzuron, chlorfluazuron, hexaflumuron, triflumuron, flufenoxuron, furcycloxuron, buprofezin, pyriproxifen, methoprene, benzoepin, diafenthiuron, fipronil, nicotine sulfate, rotenone, metaldehyde, acetamiprid, chlorphenapyl, nitenpyram, thiacloprid, clothianidin, thiamethoxam, dinotefuran, indoxacarb, pymetrozine, spinosad, emamectin, pyridalyl, tebufenozide, chromafenozide, methoxyfenozide, tolfenpyrad, flubendiamide, chlorantraniliprole, cyantraniliprole; Nematicides such as fenamiphos, phosthiazate, cadusafos; miticides such as chlorbenzilate, phenisobromolate, dicofol, amitraz, BPPS, benzomate, hexythiazox, fenbutatin-oxide, polynactin, chinomethionate, CPCBS, tetradifon, avermectin, milbemectin, clofentezine, cyhexatin, pyridaben, fenpyroximate, tebufenpyrad, cyenopyrafen, cyflumetofen, pyrimidifen, phenothiocarb, dienochlor, fluacrypyrim, acequinocyl, bifenazate, etoxazole, spirodiclofen, fenazaquin; microorganism-derived formulations such as BT agents; and the like.

Among these, particularly preferred are neonicotinoid based insecticides such as imidacloprid, acetamiprid, nitenpyram, thiacloprid, clothianidin, thiamethoxam, dinotefuran, nithiazine; and insecticides or miticides which have respiratory inhibition effects such as chlorphenapyl, pymetrozine, pyridaben, fenpyroximate, tolfenpyrad, tebufenpyrad, cyenopyrafen, cyflumetofen, fluacrypyrim, acequinocyl, fenazaquin.

Examples of plant hormones include gibberellins (for example, gibberellin A3, gibberellin A4, gibberellin A7 and the like), auxins (for example, 2,4-D, IAA, NAA and the like), cytokinins (for example, kinetin, benzyladenine and the like), abscisic acid, jasmone acids, brassinosteroids, strigolactones, salicylic acid and the like.

As plant growth regulators, in addition to the aforementioned plant hormones, mentioned are hymexazol, uniconazole, trinexapac, daminozide, cyanamide and the like.

Examples of fertilizers include nitrogenous fertilizers, phosphatic fertilizers, potash fertilizers, calcareous fertilizers, magnesium fertilizers, silicate fertilizers, trace element fertilizers, animal matter fertilizers, plant matter fertilizers and the like. When the concentration of a water soluble component of a fertilizer is too high, fertilizer disorders such as withering and death of root and leaf may be caused to a plant. Further, when a certain type of a fertilizer such as ammonium sulfate is used in a large amount, the growth of a plant may be compromised through soil acidification.

A surfactant is used as an auxiliary component of an agrochemical formulation, as an active component of some insecticides or miticides, or as a spreader. Examples of surfactants include nonionic surfactants such alkylphenyl

ether in which polyoxyethylene is added, alkyl ether in which polyoxyethylene is added, higher fatty acid ester in which polyoxyethylene is added, sorbitan higher fatty acid ester in which polyoxyethylene is added, tristyrylphenyl ether in which polyoxyethylene added; anionic surfactants such as a sulfuric ester salt of alkylphenyl ether in which polyoxyethylene is added, alkylbenzene sulfonate, a sulfuric ester salt of higher alcohol, alkylnaphthalenesulfonate, polycarboxylate, lignin sulfonate, a formaldehyde condensate of alkylnaphthalenesulfonate, a copolymer of isobutylene-maleic anhydride; cationic surfactants such as alkyltrimethylammonium chloride, methyl·polyoxyethylene·alkylammonium chloride, alkyl·*N*-methylpyridium bromide, mono- or di-alkylmethylated ammonium chloride, alkylpentamethylpropylenediamine dichloride, alkyldimethylbenzalkonium chloride, benzethonium chloride; amphoteric surfactants such as dialkyldiaminoethylbetaine, alkyldimethylbenzylbetaine, dialkyldiaminoethylglycine, alkyldimethylbenzylglycine; and the like.

EXAMPLES

The present invention will be described in detail with reference to Examples, but the scope of the present invention shall not be limited by these.

Various substances (A) were synthesized by esterifying, glycosylating or oxidizing ascorbic acid, isoascorbic acid or dehydroascorbic acid by a known reaction. Some of the substances (A) synthesized are shown in Tables 1 and 2. $R^1$ to $R^4$ in Table 1 correspond to $R^1$ to $R^4$ in Formula (I). $R^5$ and $R^6$ in Table 2 correspond to $R^5$ and $R^6$ in Formula (II).

[Table 1]

Table 1

| Compound # | $R^1$ | $R^2$ | $R^3$ | $R^4$ |
|---|---|---|---|---|
| 1 | H | H | H | H |
| 2 | $SO_3H$ | H | H | H |
| 3 | $PO_3H_2$ | H | H | H |
| 4 | glucosyl | H | H | H |
| 5 | mannosyl | H | H | H |
| 6 | galactosyl | H | H | H |
| 7 | $COCH_3$ | H | H | H |
| 8 | $COC_3H_7$-i | H | H | H |
| 9 | $COC_{17}H_{35}$-n | H | H | H |
| 10 | $COC_{16}H_{33}$-n | H | H | H |
| 11 | $COC_{18}H_{37}$-n | H | H | H |
| 12 | $CO(CH_2)_7CH=CHC_6H_{13}$-n | H | H | H |
| 13 | $COCH=CH_2$ | H | H | H |
| 14 | $COCH_2CH=CH_2$ | H | H | H |
| 15 | H | $SO_3H$ | H | H |
| 16 | H | $PO_3H_2$ | H | H |
| 17 | H | glucosyl | H | H |
| 18 | H | mannosyl | H | H |
| 19 | H | galactosyl | H | H |
| 20 | H | $COCH_3$ | H | H |
| 21 | H | $COC_3H_7$-i | H | H |
| 22 | H | $COC_{17}H_{35}$-n | H | H |
| 23 | H | $COC_{16}H_{33}$-n | H | H |
| 24 | H | $COC_{18}H_{37}$-n | H | H |
| 25 | H | $CO(CH_2)_7CH=CHC_6H_{13}$-n | H | H |

(continued)

| Compound # | $R^1$ | $R^2$ | $R^3$ | $R^4$ |
|---|---|---|---|---|
| 26 | H | $COCH=CH_2$ | H | H |
| 27 | H | $COCH_2CH=CH_2$ | H | H |
| 28 | H | H | $SO_3H$ | H |
| 29 | H | H | $PO_3H_2$ | H |
| 30 | H | H | glucosyl | H |
| 31 | H | H | mannosyl | H |
| 32 | H | H | galactosyl | H |
| 33 | H | H | $COCH_3$ | H |
| 34 | H | H | $COC_3H_7$-i | H |
| 35 | H | H | $COC_{17}H_{35}$-n | H |
| 36 | H | H | $COC_{16}H_{33}$-n | H |
| 37 | H | H | $COC_{18}H_{37}$-n | H |
| 38 | H | H | $CO(CH_2)_7CH=CHC_6H_{13}$-n | H |
| 39 | H | H | $COCH=CH_2$ | H |
| 40 | H | H | $COCH_2CH=CH_2$ | H |
| 41 | H | H | H | $SO_3H$ |
| 42 | H | H | H | $PO_3H_2$ |
| 43 | H | H | H | glucosyl |
| 44 | H | H | H | mannosyl |
| 45 | H | H | H | galactosyl |
| 46 | H | H | H | $COCH_3$ |
| 47 | H | H | H | $COC_3H_7$-i |
| 48 | H | H | H | $COC_{17}H_{35}$-n |
| 49 | H | H | H | $COC_{16}H_{33}$-n |
| 50 | H | H | H | $COC_{18}H_{37}$-n |

(continued)

| Compound # | $R^1$ | $R^2$ | $R^3$ | $R^4$ |
|---|---|---|---|---|
| 51 | H | H | H | $CO(CH_2)_7CH=CHC_6H_{13}$-n |
| 52 | H | H | H | $COCH=CH_2$ |
| 53 | H | H | H | $COCH_2CH=CH_2$ |
| 54 | $SO_3H$ | $SO_3H$ | H | H |
| 55 | $SO_3H$ | $PO_3H_2$ | H | H |
| 56 | $SO_3H$ | glucosyl | H | H |
| 57 | $SO_3H$ | mannosyl | H | H |
| 58 | $SO_3H$ | galactosyl | H | H |
| 59 | $SO_3H$ | $COCH_3$ | H | H |
| 60 | $SO_3H$ | $COC_3H_7$-i | H | H |
| 61 | $SO_3H$ | $COC_{17}H_{35}$-n | H | H |
| 62 | $SO_3H$ | $COC_{16}H_{33}$-n | H | H |
| 63 | $SO_3H$ | $COC_{18}H_{37}$-n | H | H |
| 64 | $SO_3H$ | $CO(CH_2)_7CH=CHC_6H_{13}$-n | H | H |
| 65 | $SO_3H$ | $COCH=CH_2$ | H | H |
| 66 | $SO_3H$ | $COCH_2CH=CH_2$ | H | H |
| 67 | $SO_3H$ | $SO_3H$ | H | H |
| 68 | $SO_3H$ | $PO_3H_2$ | H | H |
| 69 | $SO_3H$ | glucosyl | H | H |
| 70 | $SO_3H$ | mannosyl | H | H |
| 71 | $SO_3H$ | galactosyl | H | H |
| 72 | $SO_3H$ | $COCH_3$ | H | H |
| 73 | $SO_3H$ | $COC_3H_7$-i | H | H |
| 74 | $SO_3H$ | $COC_{17}H_{35}$-n | H | H |
| 75 | $SO_3H$ | $COC_{16}H_{33}$-n | H | H |

(continued)

| Compound # | $R^1$ | $R^2$ | $R^3$ | $R^4$ |
|---|---|---|---|---|
| 76 | $SO_3H$ | $COC_{18}H_{37}$-n | H | H |
| 77 | $SO_3H$ | $CO(CH_2)_7CH=CHC_6H_{13}$-n | H | H |
| 78 | $SO_3H$ | $COCH=CH_2$ | H | H |
| 79 | $SO_3H$ | $COCH_2CH=CH_2$ | H | H |
| 80 | glucosyl | $SO_3H$ | H | H |
| 81 | glucosyl | $PO_3H_2$ | H | H |
| 82 | glucosyl | glucosyl | H | H |
| 83 | glucosyl | mannosyl | H | H |
| 84 | glucosyl | galactosyl | H | H |
| 85 | glucosyl | $COCH_3$ | H | H |
| 86 | glucosyl | $COC_3H_7$-i | H | H |
| 87 | glucosyl | $COC_{17}H_{35}$-n | H | H |
| 88 | glucosyl | $COC_{16}H_{33}$-n | H | H |
| 89 | glucosyl | $COC_{18}H_{37}$-n | H | H |
| 90 | glucosyl | $CO(CH_2)_7CH=CHC_6H_{13}$-n | H | H |
| 91 | glucosyl | $COCH=CH_2$ | H | H |
| 92 | glucosyl | $COCH_2CH=CH_2$ | H | H |
| 93 | $COC_{16}H_{33}$ | $SO_3H$ | H | H |
| 94 | $COC_{16}H_{33}$ | $PO_3H_2$ | H | H |
| 95 | $COC_{16}H_{33}$ | glucosyl | H | H |
| 96 | $COC_{16}H_{33}$ | mannosyl | H | H |
| 97 | $COC_{16}H_{33}$ | galactosyl | H | H |
| 98 | $COC_{16}H_{33}$ | $COCH_3$ | H | H |
| 99 | $COC_{16}H_{33}$ | $COC_3H_7$-i | H | H |
| 100 | $COC_{16}H_{33}$ | $COC_{17}H_{35}$-n | H | H |

(continued)

| Compound # | $R^1$ | $R^2$ | $R^3$ | $R^4$ |
|---|---|---|---|---|
| 101 | $COC_{16}H_{33}$ | $COC_{16}H_{33}$-n | H | H |
| 102 | $COC_{16}H_{33}$ | $COC_{18}H_{37}$-n | H | H |
| 103 | $COC_{16}H_{33}$ | $CO(CH_2)_7CH=CHC_6H_{13}$-n | H | H |
| 104 | $COC_{16}H_{33}$ | $COCH=CH_2$ | H | H |
| 105 | $COC_{16}H_{33}$ | $COCH_2CH=CH_2$ | H | H |
| 106 | $CO(CHC)_7CH=CHC_6H_{13}$ | $SO_3H$ | H | H |
| 107 | $CO(CH_2)_7CH=CHC_6H_{13}$ | $PO_3H_2$ | H | H |
| 108 | $CO(CH_2)_7CH=CHC_6H_{13}$ | glucosyl | H | H |
| 109 | $CO(CH_2)_7CH=CHC_6H_{13}$ | mannosyl | H | H |
| 110 | $CO(CH_2)_7CH=CHC_6H_{13}$ | galactosyl | H | H |
| 111 | $CO(CH_2)_7CH=CHC_6H_{13}$ | $COCH_3$ | H | H |
| 112 | $CO(CH_2)_7CH=CHC_6H_{13}$ | $COC_3H_7$-i | H | H |
| 113 | $CO(CH_2)_7CH=CHC_6H_{13}$ | $COC_{17}H_{35}$-n | H | H |
| 114 | $CO(CH_2)_7CH=CHC_6H_{13}$ | $COC_{16}H_{33}$-n | H | H |
| 115 | $CO(CH_2)_7CH=CHC_6H_{13}$ | $COC_{18}H_{37}$-n | H | H |
| 116 | $CO(CH_2)_7CH=CHC_6H_{13}$ | $CO(CH_2)_7CH=CHC_6H_{13}$-n | H | H |
| 117 | $CO(CH_2)_7CH=CHC_6H_{13}$ | $COCH=CH_2$ | H | H |
| 118 | $CO(CH_2)_7CH=CHC_6H_{13}$ | $COCH_2CH=CH_2$ | H | H |
| 119 | $SO_3H$ | H | $SO_3H$ | H |
| 120 | $SO_3H$ | H | $PO_3H_2$ | H |
| 121 | $SO_3H$ | H | glucosyl | H |
| 122 | $SO_3H$ | H | mannosyl | H |
| 123 | $SO_3H$ | H | galactosyl | H |
| 124 | $SO_3H$ | H | $COCH_3$ | H |
| 125 | $SO_3H$ | H | $COC_3H_7$-i | H |

(continued)

| Compound # | $R^1$ | $R^2$ | $R^3$ | $R^4$ |
|---|---|---|---|---|
| 126 | $SO_3H$ | H | $COC_{17}H_{35}$-n | H |
| 127 | $SO_3H$ | H | $COC_{16}H_{33}$-n | H |
| 128 | $SO_3H$ | H | $COC_{18}H_{37}$-n | H |
| 129 | $SO_3H$ | H | $CO(CH_2)_7CH=CHC_6H_{13}$-n | H |
| 130 | $SO_3H$ | H | $COCH=CH_2$ | H |
| 131 | $SO_3H$ | H | $COCH_2CH=CH_2$ | H |
| 132 | $PO_3H_2$ | H | $SO_3H$ | H |
| 133 | $PO_3H_2$ | H | $PO_3H_2$ | H |
| 134 | $PO_3H_2$ | H | glucosyl | H |
| 135 | $PO_3H_2$ | H | mannosyl | H |
| 136 | $PO_3H_2$ | H | galactosyl | H |
| 137 | $PO_3H_2$ | H | $COCH_3$ | H |
| 138 | $PO_3H_2$ | H | $COC_3H_7$-i | H |
| 139 | $PO_3H_2$ | H | $COC_{17}H_{35}$-n | H |
| 140 | $PO_3H_2$ | H | $COC_{16}H_{33}$-n | H |
| 141 | $PO_3H_2$ | H | $COC_{18}H_{37}$-n | H |
| 142 | $PO_3H_2$ | H | $CO(CH_2)_7CH=CHC_6H_{13}$-n | H |
| 143 | $PO_3H_2$ | H | $COCH=CH_2$ | H |
| 144 | $PO_3H_2$ | H | $COCH_2CH=CH_2$ | H |
| 145 | glucosyl | H | $SO_3H$ | H |
| 146 | glucosyl | H | $PO_3H_2$ | H |
| 147 | glucosyl | H | glucosyl | H |
| 148 | glucosyl | H | mannosyl | H |
| 149 | glucosyl | H | galactosyl | H |
| 150 | glucosyl | H | $COCH_3$ | H |

(continued)

| Compound # | $R^1$ | $R^2$ | $R^3$ | $R^4$ |
|---|---|---|---|---|
| 151 | glucosyl | H | $COC_3H_7$-i | H |
| 152 | glucosyl | H | $COC_{17}H_{35}$-n | H |
| 153 | glucosyl | H | $COC_{16}H_{33}$-n | H |
| 154 | glucosyl | H | $COC_{18}H_{37}$-n | H |
| 155 | glucosyl | H | $CO(CH_2)_7CH{=}CHC_6H_{13}$-n | H |
| 156 | glucosyl | H | $COCH{=}CH_2$ | H |
| 157 | glucosyl | H | $COCH_2CH{=}CH_2$ | H |
| 158 | $COC_{16}H_{33}$-n | H | $SO_3H$ | H |
| 159 | $COC_{16}H_{33}$-n | H | $PO_3H_2$ | H |
| 160 | $COC_{16}H_{33}$-n | H | glucosyl | H |
| 161 | $COC_{16}H_{33}$-n | H | mannosyl | H |
| 162 | $COC_{16}H_{33}$-n | H | galactosyl | H |
| 163 | $COC_{16}H_{33}$-n | H | $COCH_3$ | H |
| 164 | $COC_{16}H_{33}$-n | H | $COC_3H_7$-i | H |
| 165 | $COC_{16}H_{33}$-n | H | $COC_{17}H_{35}$-n | H |
| 166 | $COC_{16}H_{33}$-n | H | $COC_{16}H_{33}$-n | H |
| 167 | $COC_{16}H_{33}$-n | H | $COC_{18}H_{37}$-n | H |
| 168 | $COC_{16}H_{33}$-n | H | $CO(CH_2)_7CH{=}CHC_6H_{13}$-n | H |
| 169 | $COC_{16}H_{33}$-n | H | $COCH{=}CH_2$ | H |
| 170 | $COC_{16}H_{33}$-n | H | $COCH_2CH{=}CH_2$ | H |
| 171 | $CO(CH_2)_7CH{=}CHC_6H_{13}$-n | H | $SO_3H$ | H |
| 172 | $CO(CH_2)_7CH{=}CHC_6H_{13}$-n | H | $PO_3H_2$ | H |
| 173 | $CO(CH_2)_7CH{=}CHC_6H_{13}$-n | H | glucosyl | H |
| 174 | $CO(CH_2)_7CH{=}CHC_6H_{13}$-n | H | mannosyl | H |
| 175 | $CO(CH_2)_7CH{=}CHC_6H_{13}$-n | H | galactosyl | H |

(continued)

| Compound # | $R^1$ | $R^2$ | $R^3$ | $R^4$ |
|---|---|---|---|---|
| 176 | $CO(CH_2)_7CH=CHC_6H_{13}$-n | H | $COCH_3$ | H |
| 177 | $CO(CH_2)_7CH=CHC_6H_{13}$-n | H | $COC_3H_7$-i | H |
| 178 | $CO(CH_2)_7CH=CHC_6H_{13}$-n | H | $COC_{17}H_{35}$-n | H |
| 179 | $CO(CH_2)_7CH=CHC_6H_{13}$-n | H | $COC_{16}H_{33}$-n | H |
| 180 | $CO(CH_2)_7CH=CHC_6H_{13}$-n | H | $COC_{18}H_{37}$-n | H |
| 181 | $CO(CH_2)_7CH=CHC_6H_{13}$-n | H | $CO(CH_2)_7CH=CHC_6H_{13}$-n | H |
| 182 | $CO(CH_2)_7CH=CHC_6H_{13}$-n | H | $COCH=CH_2$ | H |
| 183 | $CO(CH_2)_7CH=CHC_6H_{13}$-n | H | $COCH_2CH=CH_2$ | H |
| 184 | $SO_3H$ | H | H | $SO_3H$ |
| 185 | $SO_3H$ | H | H | $PO_3H_2$ |
| 186 | $SO_3H$ | H | H | glucosyl |
| 187 | $SO_3H$ | H | H | mannosyl |
| 188 | $SO_3H$ | H | H | galactosyl |
| 189 | $SO_3H$ | H | H | $COCH_3$ |
| 190 | $SO_3H$ | H | H | $COC_3H_7$-i |
| 191 | $SO_3H$ | H | H | $COC_{17}H_{35}$-n |
| 192 | $SO_3H$ | H | H | $COC_{16}H_{33}$-n |
| 193 | $SO_3H$ | H | H | $COC_{18}H_{37}$-n |
| 194 | $SO_3H$ | H | H | $CO(CH_2)_7CH=CHC_6H_{13}$-n |
| 195 | $SO_3H$ | H | H | $COCH=CH_2$ |
| 196 | $SO_3H$ | H | H | $COCH_2CH=CH_2$ |
| 197 | $PO_3H_2$ | H | H | $SO_3H$ |
| 198 | $PO_3H_2$ | H | H | $PO_3H_2$ |
| 199 | $PO_3H_2$ | H | H | glucosyl |
| 200 | $PO_3H_2$ | H | H | mannosyl |

(continued)

| Compound # | $R^1$ | $R^2$ | $R^3$ | $R^4$ |
|---|---|---|---|---|
| 201 | $PO_3H_2$ | H | H | galactosyl |
| 202 | $PO_3H_2$ | H | H | $COCH_3$ |
| 203 | $PO_3H_2$ | H | H | $COC_3H_7$-i |
| 204 | $PO_3H_2$ | H | H | $COC_{17}H_{35}$-n |
| 205 | $PO_3H_2$ | H | H | $COC_{16}H_{33}$-n |
| 206 | $PO_3H_2$ | H | H | $COC_{18}H_{37}$-n |
| 207 | $PO_3H_2$ | H | H | $CO(CH_2)_7CH{=}CHC_6H_{13}$-n |
| 208 | $PO_3H_2$ | H | H | $COCH{=}CH_2$ |
| 209 | $PO_3H_2$ | H | H | $COCH_2CH{=}CH_2$ |
| 210 | glucosyl | H | H | $SO_3H$ |
| 211 | glucosyl | H | H | $PO_3H_2$ |
| 212 | glucosyl | H | H | glucosyl |
| 213 | glucosyl | H | H | mannosyl |
| 214 | glucosyl | H | H | galactosyl |
| 215 | glucosyl | H | H | $COCH_3$ |
| 216 | glucosyl | H | H | $COC_3H_7$-i |
| 217 | glucosyl | H | H | $COC_{17}H_{35}$-n |
| 218 | glucosyl | H | H | $COC_{16}H_{33}$-n |
| 219 | glucosyl | H | H | $COC_{18}H_{37}$-n |
| 220 | glucosyl | H | H | $CO(CH_2)_7CH{=}CHC_6H_{13}$-n |
| 221 | glucosyl | H | H | $COCH{=}CH_2$ |
| 222 | glucosyl | H | H | $COCH_2CH{=}CH_2$ |
| 223 | $COC_{16}H_{33}$-n | H | H | $SO_3H$ |
| 224 | $COC_{16}H_{33}$-n | H | H | $PO_3H_2$ |
| 225 | $COC_{16}H_{33}$-n | H | H | glucosyl |

(continued)

| Compound # | $R^1$ | $R^2$ | $R^3$ | $R^4$ |
|---|---|---|---|---|
| 226 | $COC_{16}H_{33}$-n | H | H | mannosyl |
| 227 | $COC_{16}H_{33}$-n | H | H | galactosyl |
| 228 | $COC_{16}H_{33}$-n | H | H | $COCH_3$ |
| 229 | $COC_{16}H_{33}$-n | H | H | $COC_3H_7$-i |
| 230 | $COC_{16}H_{33}$-n | H | H | $COC_{17}H_{35}$-n |
| 231 | $COC_{16}H_{33}$-n | H | H | $COC_{16}H_{33}$-n |
| 232 | $COC_{16}H_{33}$-n | H | H | $COC_{18}H_{37}$-n |
| 233 | $COC_{16}H_{33}$-n | H | H | $CO(CH_2)_7CH=CHC_6H_{13}$-n |
| 234 | $COC_{16}H_{33}$-n | H | H | $COCH=CH_2$ |
| 235 | $COC_{16}H_{33}$-n | H | H | $COCH_2CH=CH_2$ |
| 236 | $CO(CH_2)_7CH=CHC_6H_{13}$-n | H | H | $SO_3H$ |
| 237 | $CO(CH_2)_7CH=CHC_6H_{13}$-n | H | H | $PO_3H_2$ |
| 238 | $CO(CH_2)_7CH=CHC_6H_{13}$-n | H | H | glucosyl |
| 239 | $CO(CH_2)_7CH=CHC_6H_{13}$-n | H | H | mannosyl |
| 240 | $CO(CH_2)_7CH=CHC_6H_{13}$-n | H | H | galactosyl |
| 241 | $CO(CH_2)_7CH=CHC_6H_{13}$-n | H | H | $COCH_3$ |
| 242 | $CO(CH_2)_7CH=CHC_6H_{13}$-n | H | H | $COC_3H_7$-i |
| 243 | $CO(CH_2)_7CH=CHC_6H_{13}$-n | H | H | $COC_{17}H_{35}$-n |
| 244 | $CO(CH_2)_7CH=CHC_6H_{13}$-n | H | H | $COC_{16}H_{33}$-n |
| 245 | $CO(CH_2)_7CH=CHC_6H_{13}$-n | H | H | $COC_{18}H_{37}$-n |
| 246 | $CO(CH_2)_7CH=CHC_6H_{13}$-n | H | H | $CO(CH_2)_7CH=CHC_6H_{13}$-n |
| 247 | $CO(CH_2)_7CH=CHC_6H_{13}$-n | H | H | $COCH=CH_2$ |
| 248 | $CO(CH_2)_7CH=CHC_6H_{13}$-n | H | H | $COCH_2CH=CH_2$ |
| 249 | $SO_3H$ | $SO_3H$ | $SO_3H$ | H |
| 250 | $SO_3H$ | $SO_3H$ | $PO_3H_2$ | H |

(continued)

| Compound # | $R^1$ | $R^2$ | $R^3$ | $R^4$ |
|---|---|---|---|---|
| 251 | $SO_3H$ | $SO_3H$ | glucosyl | H |
| 252 | $SO_3H$ | $SO_3H$ | mannosyl | H |
| 253 | $SO_3H$ | $SO_3H$ | galactosyl | H |
| 254 | $SO_3H$ | $SO_3H$ | $COCH_3$ | H |
| 255 | $SO_3H$ | $SO_3H$ | $COC_3H_7$-i | H |
| 256 | $SO_3H$ | $SO_3H$ | $COC_{17}H_{35}$-n | H |
| 257 | $SO_3H$ | $SO_3H$ | $COC_{16}H_{33}$-n | H |
| 258 | $SO_3H$ | $SO_3H$ | $COC_{18}H_{37}$-n | H |
| 259 | $SO_3H$ | $SO_3H$ | $CO(CH_2)_7CH=CHC_6H_{13}$-n | H |
| 260 | $SO_3H$ | $SO_3H$ | $COCH=CH_2$ | H |
| 261 | $SO_3H$ | $SO_3H$ | $COCH_2CH=CH_2$ | H |
| 262 | $PO_3H_2$ | $PO_3H_2$ | $SO_3H$ | H |
| 263 | $PO_3H_2$ | $PO_3H_2$ | $PO_3H_2$ | H |
| 264 | $PO_3H_2$ | $PO_3H_2$ | glucosyl | H |
| 265 | $PO_3H_2$ | $PO_3H_2$ | mannosyl | H |
| 266 | $PO_3H_2$ | $PO_3H_2$ | galactosyl | H |
| 267 | $PO_3H_2$ | $PO_3H_2$ | $COCH_3$ | H |
| 268 | $PO_3H_2$ | $PO_3H_2$ | $COC_3H_7$-i | H |
| 269 | $PO_3H_2$ | $PO_3H_2$ | $COC_{17}H_{35}$-n | H |
| 270 | $PO_3H_2$ | $PO_3H_2$ | $COC_{16}H_{33}$-n | H |
| 271 | $PO_3H_2$ | $PO_3H_2$ | $COC_{18}H_{37}$-n | H |
| 272 | $PO_3H_2$ | $PO_3H_2$ | $CO(CH_2)_7CH=CHC_6H_{13}$-n | H |
| 273 | $PO_3H_2$ | $PO_3H_2$ | $COCH=CH_2$ | H |
| 274 | $PO_3H_2$ | $PO_3H_2$ | $COCH_2CH=CH_2$ | H |
| 275 | glucosyl | glucosyl | $SO_3H$ | H |

(continued)

| Compound # | $R^1$ | $R^2$ | $R^3$ | $R^4$ |
|---|---|---|---|---|
| 276 | glucosyl | glucosyl | $PO_3H_2$ | H |
| 277 | glucosyl | glucosyl | glucosyl | H |
| 278 | glucosyl | glucosyl | mannosyl | H |
| 279 | glucosyl | glucosyl | galactosyl | H |
| 280 | glucosyl | glucosyl | $COCH_3$ | H |
| 281 | glucosyl | glucosyl | $COC_3H_7$-i | H |
| 282 | glucosyl | glucosyl | $COC_{17}H_{35}$-n | H |
| 283 | glucosyl | glucosyl | $COC_{16}H_{33}$-n | H |
| 284 | glucosyl | glucosyl | $COC_{18}H_{37}$-n | H |
| 285 | glucosyl | glucosyl | $CO(CH_2)_7CH=CHC_6H_{13}$-n | H |
| 286 | glucosyl | glucosyl | $COCH=CH_2$ | H |
| 287 | glucosyl | glucosyl | $COCH_2CH=CH_2$ | H |
| 288 | $COC_{16}H_{33}$-n | $COC_{16}H_{33}$-n | $SO_3H$ | H |
| 289 | $COC_{16}H_{33}$-n | $COC_{16}H_{33}$-n | $PO_3H_2$ | H |
| 290 | $COC_{16}H_{33}$-n | $COC_{16}H_{33}$-n | glucosyl | H |
| 291 | $COC_{16}H_{33}$-n | $COC_{16}H_{33}$-n | mannosyl | H |
| 292 | $COC_{16}H_{33}$-n | $COC_{16}H_{33}$-n | galactosyl | H |
| 293 | $COC_{16}H_{33}$-n | $COC_{16}H_{33}$-n | $COCH_3$ | H |
| 294 | $COC_{16}H_{33}$-n | $COC_{16}H_{33}$-n | $COC_3H_7$-i | H |
| 295 | $COC_{16}H_{33}$-n | $COC_{16}H_{33}$-n | $COC_{17}H_{35}$-n | H |
| 296 | $COC_{16}H_{33}$-n | $COC_{16}H_{33}$-n | $COC_{16}H_{33}$-n | H |
| 297 | $COC_{16}H_{33}$-n | $COC_{16}H_{33}$-n | $COC_{18}H_{37}$-n | H |
| 298 | $COC_{16}H_{33}$-n | $COC_{16}H_{33}$-n | $CO(CH_2)_7CH=CHC_6H_{13}$-n | H |
| 299 | $COC_{16}H_{33}$-n | $COC_{16}H_{33}$-n | $COCH=CH_2$ | H |
| 300 | $COC_{16}H_{33}$-n | $COC_{16}H_{33}$-n | $COCH_2CH=CH_2$ | H |

(continued)

| Compound # | $R^1$ | $R^2$ | $R^3$ | $R^4$ |
|---|---|---|---|---|
| 301 | $CO(CH_2)_7CH=CHC_6H_{13}$-n | $CO(CH_2)_7CH=CHC_6H_{13}$-n | $SO_3H$ | H |
| 302 | $CO(CH_2)_7CH=CHC_6H_{13}$-n | $CO(CH_2)_7CH=CHC_6H_{13}$-n | $PO_3H_2$ | H |
| 303 | $CO(CH_2)_7CH=CHC_6H_{13}$-n | $CO(CH_2)_7CH=CHC_6H_{13}$-n | glucosyl | H |
| 304 | $CO(CH_2)_7CH=CHC_6H_{13}$-n | $CO(CH_2)_7CH=CHC_6H_{13}$-n | mannosyl | H |
| 305 | $CO(CH_2)_7CH=CHC_6H_{13}$-n | $CO(CH_2)_7CH=CHC_6H_{13}$-n | galactosyl | H |
| 306 | $CO(CH_2)_7CH=CHC_6H_{13}$-n | $CO(CH_2)_7CH=CHC_6H_{13}$-n | $COCH_3$ | H |
| 307 | $CO(CH_2)_7CH=CHC_6H_{13}$-n | $CO(CH_2)_7CH=CHC_6H_{13}$-n | $COC_3H_7$-i | H |
| 308 | $CO(CH_2)_7CH=CHC_6H_{13}$-n | $CO(CH_2)_7CH=CHC_6H_{13}$-n | $COC_{17}H_{35}$-n | H |
| 309 | $CO(CH_2)_7CH=CHC_6H_{13}$-n | $CO(CH_2)_7CH=CHC_6H_{13}$-n | $COC_{16}H_{33}$-n | H |
| 310 | $CO(CH_2)_7CH=CHC_6H_{13}$-n | $CO(CH_2)_7CH=CHC_6H_{13}$-n | $COC_{18}H_{37}$-n | H |
| 311 | $CO(CH_2)_7CH=CHC_6H_{13}$-n | $CO(CH_2)_7CH=CHC_6H_{13}$-n | $CO(CH_2)_7CH=CHC_6H_{13}$-n | H |
| 312 | $CO(CH_2)_7CH=CHC_6H_{13}$-n | $CO(CH_2)_7CH=CHC_6H_{13}$-n | $COCH=CH_2$ | H |
| 313 | $CO(CH_2)_7CH=CHC_6H_{13}$-n | $CO(CH_2)_7CH=CHC_6H_{13}$-n | $COCH_2CH=CH_2$ | H |
| 314 | $SO_3H$ | $SO_3H$ | H | $SO_3H$ |
| 315 | $SO_3H$ | $SO_3H$ | H | $PO_3H_2$ |
| 316 | $SO_3H$ | $SO_3H$ | H | glucosyl |
| 317 | $SO_3H$ | $SO_3H$ | H | mannosyl |
| 318 | $SO_3H$ | $SO_3H$ | H | galactosyl |
| 319 | $SO_3H$ | $SO_3H$ | H | $COCH_3$ |
| 320 | $SO_3H$ | $SO_3H$ | H | $COC_3H_7$-i |
| 321 | $SO_3H$ | $SO_3H$ | H | $COC_{17}H_{35}$-n |
| 322 | $SO_3H$ | $SO_3H$ | H | $COC_{16}H_{33}$-n |
| 323 | $SO_3H$ | $SO_3H$ | H | $COC_{18}H_{37}$-n |
| 324 | $SO_3H$ | $SO_3H$ | H | $CO(CH_2)_7CH=CHC_6H_{13}$-n |
| 325 | $SO_3H$ | $SO_3H$ | H | $COCH=CH_2$ |

(continued)

| Compound # | $R^1$ | $R^2$ | $R^3$ | $R^4$ |
|---|---|---|---|---|
| 326 | $SO_3H$ | $SO_3H$ | H | $COCH_2CH=CH_2$ |
| 327 | $PO_3H_2$ | $PO_3H_2$ | H | $SO_3H$ |
| 328 | $PO_3H_2$ | $PO_3H_2$ | H | $PO_3H_2$ |
| 329 | $PO_3H_2$ | $PO_3H_2$ | H | glucosyl |
| 330 | $PO_3H_2$ | $PO_3H_2$ | H | mannosyl |
| 331 | $PO_3H_2$ | $PO_3H_2$ | H | galactosyl |
| 332 | $PO_3H_2$ | $PO_3H_2$ | H | $COCH_3$ |
| 333 | $PO_3H_2$ | $PO_3H_2$ | H | $COC_3H_7$-i |
| 334 | $PO_3H_2$ | $PO_3H_2$ | H | $COC_{17}H_{35}$-n |
| 335 | $PO_3H_2$ | $PO_3H_2$ | H | $COC_{16}H_{33}$-n |
| 336 | $PO_3H_2$ | $PO_3H_2$ | H | $COC_{18}H_{37}$-n |
| 337 | $PO_3H_2$ | $PO_3H_2$ | H | $CO(CH_2)_7CH=CHC_6H_{13}$-n |
| 338 | $PO_3H_2$ | $PO_3H_2$ | H | $COCH=CH_2$ |
| 339 | $PO_3H_2$ | $PO_3H_2$ | H | $COCH_2CH=CH_2$ |
| 340 | glucosyl | glucosyl | H | $SO_3H$ |
| 341 | glucosyl | glucosyl | H | $PO_3H_2$ |
| 342 | glucosyl | glucosyl | H | glucosyl |
| 343 | glucosyl | glucosyl | H | mannosyl |
| 344 | glucosyl | glucosyl | H | galactosyl |
| 345 | glucosyl | glucosyl | H | $COCH_3$ |
| 346 | glucosyl | glucosyl | H | $COC_3H_7$-i |
| 347 | glucosyl | glucosyl | H | $COC_{17}H_{35}$-n |
| 348 | glucosyl | glucosyl | H | $COC_{16}H_{33}$-n |
| 349 | glucosyl | glucosyl | H | $COC_{18}H_{37}$-n |
| 350 | glucosyl | glucosyl | H | $CO(CH_2)_7CH=CHC_6H_{13}$-n |

(continued)

| Compound # | $R^1$ | $R^2$ | $R^3$ | $R^4$ |
|---|---|---|---|---|
| 351 | glucosyl | glucosyl | H | $COCH=CH_2$ |
| 352 | glucosyl | glucosyl | H | $COCH_2CH=CH_2$ |
| 353 | $COC_{16}H_{33}$-n | $COC_{16}H_{33}$-n | H | $SO_3H$ |
| 354 | $COC_{16}H_{33}$-n | $COC_{16}H_{33}$-n | H | $PO_3H_2$ |
| 355 | $COC_{16}H_{33}$-n | $COC_{16}H_{33}$-n | H | glucosyl |
| 356 | $COC_{16}H_{33}$-n | $COC_{16}H_{33}$-n | H | mannosyl |
| 357 | $COC_{16}H_{33}$-n | $COC_{16}H_{33}$-n | H | galactosyl |
| 358 | $COC_{16}H_{33}$-n | $COC_{16}H_{33}$-n | H | $COCH_3$ |
| 359 | $COC_{16}H_{33}$-n | $COC_{16}H_{33}$-n | H | $COC_3H_7$-i |
| 360 | $COC_{16}H_{33}$-n | $COC_{16}H_{33}$-n | H | $COC_{17}H_{35}$-n |
| 361 | $COC_{16}H_{33}$-n | $COC_{16}H_{33}$-n | H | $COC_{16}H_{33}$-n |
| 362 | $COC_{16}H_{33}$-n | $COC_{16}H_{33}$-n | H | $COC_{18}H_{37}$-n |
| 363 | $COC_{16}H_{33}$-n | $COC_{16}H_{33}$-n | H | $CO(CH_2)_7CH=CHC_6H_{13}$-n |
| 364 | $COC_{16}H_{33}$-n | $COC_{16}H_{33}$-n | H | $COCH=CH_2$ |
| 365 | $COC_{16}H_{33}$-n | $COC_{16}H_{33}$-n | H | $COCH_2CH=CH_2$ |
| 366 | $CO(CH_2)_7CH=CHC_6H_{13}$-n | $CO(CH_2)_7CH=CHC_6H_{13}$-n | H | $SO_3H$ |
| 367 | $CO(CH_2)_7CH=CHC_6H_{13}$-n | $CO(CH_2)_7CH=CHC_6H_{13}$-n | H | $PO_3H_2$ |
| 368 | $CO(CH_2)_7CH=CHC_6H_{13}$-n | $CO(CH_2)_7CH=CHC_6H_{13}$-n | H | glucosyl |
| 369 | $CO(CH_2)_7CH=CHC_6H_{13}$-n | $CO(CH_2)_7CH=CHC_6H_{13}$-n | H | mannosyl |
| 370 | $CO(CH_2)_7CH=CHC_6H_{13}$-n | $CO(CH_2)_7CH=CHC_6H_{13}$-n | H | galactosyl |
| 371 | $CO(CH_2)_7CH=CHC_6H_{13}$-n | $CO(CH_2)_7CH=CHC_6H_{13}$-n | H | $COCH_3$ |
| 372 | $CO(CH_2)_7CH=CHC_6H_{13}$-n | $CO(CH_2)_7CH=CHC_6H_{13}$-n | H | $COC_3H_7$-i |
| 373 | $CO(CH_2)_7CH=CHC_6H_{13}$-n | $CO(CH_2)_7CH=CHC_6H_{13}$-n | H | $COC_{17}H_{35}$-n |
| 374 | $CO(CH_2)_7CH=CHC_6H_{13}$-n | $CO(CH_2)_7CH=CHC_6H_{13}$-n | H | $COC_{16}H_{33}$-n |
| 375 | $CO(CH_2)_7CH=CHC_6H_{13}$-n | $CO(CH_2)_7CH=CHC_6H_{13}$-n | H | $COC_{18}H_{37}$-n |

(continued)

| Compound # | $R^1$ | $R^2$ | $R^3$ | $R^4$ |
|---|---|---|---|---|
| 376 | $CO(CH_2)_7CH{=}CHC_6H_{13}$-n | $CO(CH_2)_7CH{=}CHC_6H_{13}$-n | H | $CO(CH_2)_7CH{=}CHC_6H_{13}$-n |
| 377 | $CO(CH_2)_7CH{=}CHC_6H_{13}$-n | $CO(CH_2)_7CH{=}CHC_6H_{13}$-n | H | $COCH{=}CH_2$ |
| 378 | $CO(CH_2)_7CH{=}CHC_6H_{13}$-n | $CO(CH_2)_7CH{=}CHC_6H_{13}$-n | H | $COCH_2CH{=}CH_2$ |
| 379 | $SO_3H$ | $SO_3H$ | $SO_3H$ | $SO_3H$ |
| 380 | $SO_3H$ | $SO_3H$ | $SO_3H$ | $PO_3H_2$ |
| 381 | $SO_3H$ | $SO_3H$ | $SO_3H$ | glucosyl |
| 382 | $SO_3H$ | $SO_3H$ | $SO_3H$ | mannosyl |
| 383 | $SO_3H$ | $SO_3H$ | $SO_3H$ | galactosyl |
| 384 | $SO_3H$ | $SO_3H$ | $SO_3H$ | $COCH_3$ |
| 385 | $SO_3H$ | $SO_3H$ | $SO_3H$ | $COC_3H_7$-i |
| 386 | $SO_3H$ | $SO_3H$ | $SO_3H$ | $COC_{17}H_{35}$-n |
| 387 | $SO_3H$ | $SO_3H$ | $SO_3H$ | $COC_{16}H_{33}$-n |
| 388 | $SO_3H$ | $SO_3H$ | $SO_3H$ | $COC_{18}H_{37}$-n |
| 389 | $SO_3H$ | $SO_3H$ | $SO_3H$ | $CO(CH_2)_7CH{=}CHC_6H_{13}$-n |
| 390 | $SO_3H$ | $SO_3H$ | $SO_3H$ | $COCH{=}CH_2$ |
| 391 | $SO_3H$ | $SO_3H$ | $SO_3H$ | $COCH_2CH{=}CH_2$ |
| 392 | $PO_3H_2$ | $PO_3H_2$ | $PO_3H_2$ | $SO_3H$ |
| 393 | $PO_3H_2$ | $PO_3H_2$ | $PO_3H_2$ | $PO_3H_2$ |
| 394 | $PO_3H_2$ | $PO_3H_2$ | $PO_3H_2$ | glucosyl |
| 395 | $PO_3H_2$ | $PO_3H_2$ | $PO_3H_2$ | mannosyl |
| 396 | $PO_3H_2$ | $PO_3H_2$ | $PO_3H_2$ | galactosyl |
| 397 | $PO_3H_2$ | $PO_3H_2$ | $PO_3H_2$ | $COCH_3$ |
| 398 | $PO_3H_2$ | $PO_3H_2$ | $PO_3H_2$ | $COC_3H_7$-i |
| 399 | $PO_3H_2$ | $PO_3H_2$ | $PO_3H_2$ | $COC_{17}H_{35}$-n |
| 400 | $PO_3H_2$ | $PO_3H_2$ | $PO_3H_2$ | $COC_{16}H_{33}$-n |

(continued)

| Compound # | $R^1$ | $R^2$ | $R^3$ | $R^4$ |
|---|---|---|---|---|
| 401 | $PO_3H_2$ | $PO_3H_2$ | $PO_3H_2$ | $COC_{18}H_{37}$-n |
| 402 | $PO_3H_2$ | $PO_3H_2$ | $PO_3H_2$ | $CO(CH_2)_7CH=CHC_6H_{13}$-n |
| 403 | $PO_3H_2$ | $PO_3H_2$ | $PO_3H_2$ | $COCH=CH_2$ |
| 404 | $PO_3H_2$ | $PO_3H_2$ | $PO_3H_2$ | $COCH_2CH=CH_2$ |
| 405 | glucosyl | glucosyl | glucosyl | $SO_3H$ |
| 406 | glucosyl | glucosyl | glucosyl | $PO_3H_2$ |
| 407 | glucosyl | glucosyl | glucosyl | glucosyl |
| 408 | glucosyl | glucosyl | glucosyl | mannosyl |
| 409 | glucosyl | glucosyl | glucosyl | galactosyl |
| 410 | glucosyl | glucosyl | glucosyl | $COCH_3$ |
| 411 | glucosyl | glucosyl | glucosyl | $COC_3H_7$-i |
| 412 | glucosyl | glucosyl | glucosyl | $COC_{17}H_{35}$-n |
| 413 | glucosyl | glucosyl | glucosyl | $COC_{16}H_{33}$-n |
| 414 | glucosyl | glucosyl | glucosyl | $COC_{18}H_{37}$-n |
| 415 | glucosyl | glucosyl | glucosyl | $CO(CH_2)_7CH=CHC_6H_{13}$-n |
| 416 | glucosyl | glucosyl | glucosyl | $COCH=CH_2$ |
| 417 | glucosyl | glucosyl | glucosyl | $COCH_2CH=CH_2$ |
| 418 | $COC_{16}H_{33}$-n | $COC_{16}H_{33}$-n | $COC_{16}H_{33}$-n | $SO_3H$ |
| 419 | $COC_{16}H_{33}$-n | $COC_{16}H_{33}$-n | $COC_{16}H_{33}$-n | $PO_3H_2$ |
| 420 | $COC_{16}H_{33}$-n | $COC_{16}H_{33}$-n | $COC_{16}H_{33}$-n | glucosyl |
| 421 | $COC_{16}H_{33}$-n | $COC_{16}H_{33}$-n | $COC_{16}H_{33}$-n | mannosyl |
| 422 | $COC_{16}H_{33}$-n | $COC_{16}H_{33}$-n | $COC_{16}H_{33}$-n | galactosyl |
| 423 | $COC_{16}H_{33}$-n | $COC_{16}H_{33}$-n | $COC_{16}H_{33}$-n | $COCH_3$ |
| 424 | $COC_{16}H_{33}$-n | $COC_{16}H_{33}$-n | $COC_{16}H_{33}$-n | $COC_3H_7$-i |
| 425 | $COC_{16}H_{33}$-n | $COC_{16}H_{33}$-n | $COC_{16}H_{33}$-n | $COC_{17}H_{35}$-n |

(continued)

| Compound # | $R^1$ | $R^2$ | $R^3$ | $R^4$ |
|---|---|---|---|---|
| 426 | $COC_{16}H_{33}$-n | $COC_{16}H_{33}$-n | $COC_{16}H_{33}$-n | $COC_{16}H_{33}$-n |
| 427 | $COC_{16}H_{33}$-n | $COC_{16}H_{33}$-n | $COC_{16}H_{33}$-n | $COC_{18}H_{37}$-n |
| 428 | $COC_{16}H_{33}$-n | $COC_{16}H_{33}$-n | $COC_{16}H_{33}$-n | $CO(CH_2)_7CH=CHC_6H_{13}$-n |
| 429 | $COC_{16}H_{33}$-n | $COC_{16}H_{33}$-n | $COC_{16}H_{33}$-n | $COCH=CH_2$ |
| 430 | $COC_{16}H_{33}$-n | $COC_{16}H_{33}$-n | $COC_{16}H_{33}$-n | $COCH_2CH=CH_2$ |
| 431 | $CO(CH_2)_7CH=CHC_6H_{13}$-n | $CO(CH_2)_7CH=CHC_6H_{13}$-n | $CO(CH_2)_7CH=CHC_6H_{13}$-n | $SO_3H$ |
| 432 | $CO(CH_2)_7CH=CHC_6H_{13}$-n | $CO(CH_2)_7CH=CHC_6H_{13}$-n | $CO(CH_2)_7CH=CHC_6H_{13}$-n | $PO_3H_2$ |
| 433 | $CO(CH_2)_7CH=CHC_6H_{13}$-n | $CO(CH_2)_7CH=CHC_6H_{13}$-n | $CO(CH_2)_7CH=CHC_6H_{13}$-n | glucosyl |
| 434 | $CO(CH_2)_7CH=CHC_6H_{13}$-n | $CO(CH_2)_7CH=CHC_6H_{13}$-n | $CO(CH_2)_7CH=CHC_6H_{13}$-n | mannosyl |
| 435 | $CO(CH_2)_7CH=CHC_6H_{13}$-n | $CO(CH_2)_7CH=CHC_6H_{13}$-n | $CO(CH_2)_7CH=CHC_6H_{13}$-n | galactosyl |
| 436 | $CO(CH_2)_7CH=CHC_6H_{13}$-n | $CO(CH_2)_7CH=CHC_6H_{13}$-n | $CO(CH_2)_7CH=CHC_6H_{13}$-n | $COCH_3$ |
| 437 | $CO(CH_2)_7CH=CHC_6H_{13}$-n | $CO(CH_2)_7CH=CHC_6H_{13}$-n | $CO(CH_2)_7CH=CHC_6H_{13}$-n | $COC_3H_7$-i |
| 438 | $CO(CH_2)_7CH=CHC_6H_{13}$-n | $CO(CH_2)_7CH=CHC_6H_{13}$-n | $CO(CH_2)_7CH=CHC_6H_{13}$-n | $COC_{17}H_{35}$-n |
| 439 | $CO(CH_2)_7CH=CHC_6H_{13}$-n | $CO(CH_2)_7CH=CHC_6H_{13}$-n | $CO(CH_2)_7CH=CHC_6H_{13}$-n | $COC_{16}H_{33}$-n |
| 440 | $CO(CH_2)_7CH=CHC_6H_{13}$-n | $CO(CH_2)_7CH=CHC_6H_{13}$-n | $CO(CH_2)_7CH=CHC_6H_{13}$-n | $COC_{18}H_{37}$-n |
| 441 | $CO(CH_2)_7CH=CHC_6H_{13}$-n | $CO(CH_2)_7CH=CHC_6H_{13}$-n | $CO(CH_2)_7CH=CHC_6H_{13}$-n | $CO(CH_2)_7CH=CHC_6H_{13}$-n |
| 442 | $CO(CH_2)_7CH=CHC_6H_{13}$-n | $CO(CH_2)_7CH=CHC_6H_{13}$-n | $CO(CH_2)_7CH=CHC_6H_{13}$-n | $COCH=CH_2$ |
| 443 | $CO(CH_2)_7CH=CHC_6H_{13}$-n | $CO(CH_2)_7CH=CHC_6H_{13}$-n | $CO(CH_2)_7CH=CHC_6H_{13}$-n | $COCH_2CH=CH_2$ |

[Table 2]

Table 2

| Compound # | $R^5$ | $R^6$ |
|---|---|---|
| 444 | H | H |
| 445 | $SO_3H$ | H |
| 446 | $PO_3H_2$ | H |
| 447 | glucosyl | H |
| 448 | mannosyl | H |
| 449 | galactosyl | H |
| 450 | $COCH_3$ | H |
| 451 | $COC_3H_7$-i | H |
| 452 | $COC_{17}H_{35}$-n | H |
| 453 | $COC_{16}H_{33}$-n | H |
| 454 | $COC_{18}H_{37}$-n | H |
| 455 | $CO(CH_2)_7CH{=}CHC_6H_{13}$-n | H |
| 456 | $COCH{=}CH_2$ | H |
| 457 | $COCH_2CH{=}CH_2$ | H |
| 458 | H | $SO_3H$ |
| 459 | H | $PO_3H_2$ |
| 460 | H | glucosyl |
| 461 | H | mannosyl |
| 462 | H | galactosyl |
| 463 | H | $COCH_3$ |
| 464 | H | $COC_3H_7$-i |
| 465 | H | $COC_{17}H_{35}$-n |
| 466 | H | $COC_{16}H_{33}$-n |
| 467 | H | $COC_{18}H_{37}$-n |
| 468 | H | $CO(CH_2)_7CH{=}CHC_6H_{13}$-n |
| 469 | H | $COCH{=}CH_2$ |
| 470 | H | $COCH_2CH{=}CH_2$ |
| 471 | $SO_3H$ | $SO_3H$ |
| 472 | $SO_3H$ | $PO_3H_2$ |
| 473 | $SO_3H$ | glucosyl |
| 474 | $SO_3H$ | mannosyl |
| 475 | $SO_3H$ | galactosyl |
| 476 | $SO_3H$ | $COCH_3$ |
| 477 | $SO_3H$ | $COC_3H_7$-i |
| 478 | $SO_3H$ | $COC_{17}H_{35}$-n |
| 479 | $SO_3H$ | $COC_{16}H_{33}$-n |
| 480 | $SO_3H$ | $COC_{18}H_{37}$-n |

(continued)

| Compound # | $R^5$ | $R^6$ |
|---|---|---|
| 481 | $SO_3H$ | $CO(CH_2)_7CH=CHC_6H_{13}$-n |
| 482 | $SO_3H$ | $COCH=CH_2$ |
| 483 | $SO_3H$ | $COCH_2CH=CH_2$ |
| 484 | $PO_3H_2$ | $SO_3H$ |
| 485 | $PO_3H_2$ | $PO_3H_2$ |
| 486 | $PO_3H_2$ | glucosyl |
| 487 | $PO_3H_2$ | mannosyl |
| 488 | $PO_3H_2$ | galactosyl |
| 489 | $PO_3H_2$ | $COCH_3$ |
| 490 | $PO_3H_2$ | $COC_3H_7$-i |
| 491 | $PO_3H_2$ | $COC_{17}H_{35}$-n |
| 492 | $PO_3H_2$ | $COC_{16}H_{33}$-n |
| 493 | $PO_3H_2$ | $COC_{18}H_{37}$-n |
| 494 | $PO_3H_2$ | $CO(CH_2)_7CH=CHC_6H_{13}$-n |
| 495 | $PO_3H_2$ | $COCH=CH_2$ |
| 496 | $PO_3H_2$ | $COCH_2CH=CH_2$ |
| 497 | glucosyl | $SO_3H$ |
| 498 | glucosyl | $PO_3H_2$ |
| 499 | glucosyl | glucosyl |
| 500 | glucosyl | mannosyl |
| 501 | glucosyl | galactosyl |
| 502 | glucosyl | $COCH_3$ |
| 503 | glucosyl | $COC_3H_7$-i |
| 504 | glucosyl | $COC_{17}H_{35}$-n |
| 505 | glucosyl | $COC_{16}H_{33}$-n |
| 506 | glucosyl | $COC_{18}H_{37}$-n |
| 507 | glucosyl | $CO(CH_2)_7CH=CHC_6H_{13}$-n |
| 508 | glucosyl | $COCH=CH_2$ |
| 509 | glucosyl | $COCH_2CH=CH_2$ |
| 510 | $COC_{16}H_{33}$-n | $SO_3H$ |
| 511 | $COC_{16}H_{33}$-n | $PO_3H_2$ |
| 512 | $COC_{16}H_{33}$-n | glucosyl |
| 513 | $COC_{16}H_{33}$-n | mannosyl |
| 514 | $COC_{16}H_{33}$-n | galactosyl |
| 515 | $COC_{16}H_{33}$-n | $COCH_3$ |
| 516 | $COC_{16}H_{33}$-n | $COC_3H_7$-i |
| 517 | $COC_{16}H_{33}$-n | $COC_{17}H_{35}$-n |
| 518 | $COC_{16}H_{33}$-n | $COC_{16}H_{33}$-n |

(continued)

| Compound # | $R^5$ | $R^6$ |
|---|---|---|
| 519 | $COC_{16}H_{33}$-n | $COC_{18}H_{37}$-n |
| 520 | $COC_{16}H_{33}$-n | $CO(CH_2)_7CH=CHC_6H_{13}$-n |
| 521 | $COC_{16}H_{33}$-n | $COCH=CH_2$ |
| 522 | $COC_{16}H_{33}$-n | $COCH_2CH=CH_2$ |
| 523 | $CO(CH_2)_7CH=CHC_6H_{13}$-n | $SO_3H$ |
| 524 | $CO(CH_2)_7CH=CHC_6H_{13}$-n | $PO_3H_2$ |
| 525 | $CO(CH_2)_7CH=CHC_6H_{13}$-n | glucosyl |
| 526 | $CO(CH_2)_7CH=CHC_6H_{13}$-n | mannosyl |
| 527 | $CO(CH_2)_7CH=CHC_6H_{13}$-n | galactosyl |
| 528 | $CO(CH_2)_7CH=CHC_6H_{13}$-n | $COCH_3$ |
| 529 | $CO(CH_2)_7CH=CHC_6H_{13}$-n | $COC_3H_7$-i |
| 530 | $CO(CH_2)_7CH=CHC_6H_{13}$-n | $COC_{17}H_{35}$-n |
| 531 | $CO(CH_2)_7CH=CHC_6H_{13}$-n | $COC_{16}H_{33}$-n |
| 532 | $CO(CH_2)_7CH=CHC_6H_{13}$-n | $COC_{18}H_{37}$-n |
| 533 | $CO(CH_2)_7CH=CHC_6H_{13}$-n | $CO(CH_2)_7CH=CHC_6H_{13}$-n |
| 534 | $CO(CH_2)_7CH=CHC_6H_{13}$-n | $COCH=CH_2$ |
| 535 | $CO(CH_2)_7CH=CHC_6H_{13}$-n | $COCH_2CH=CH_2$ |

Next, some examples of the formulations according to the present invention are shown. There is no particular limitation for mixing prescriptions for the formulations, and they are widely modifiable. The parts in the formulations of Examples represent parts by weight.

(Formulation Example 1) Wettable powder

| | |
|---|---|
| Substance (A) | 20 parts |
| White carbon | 20 parts |
| Diatomaceous earth | 52 parts |
| Sodium alkyl sulfate | 8 parts |

The above materials are uniformly mixed, and finely ground to obtain a wettable powder.

(Formulation Example 2) Emulsifiable Concentrate

| | |
|---|---|
| Substance (A) | 20 parts |
| Xylene | 55 parts |
| Dimethylformamide | 15 parts |
| Polyoxyethylene phenyl ether | 10 parts |

The above materials are mixed, and dissolved to obtain an emulsion.

(Formulation Example 3) Granular formulation

| | |
|---|---|
| Substance (A) | 10 parts |
| Talc | 37 parts |
| Clay | 36 parts |
| Bentonite | 10 parts |
| Sodium alkyl sulfate | 7 parts |

The above materials are uniformly mixed, finely ground, and then granulated to obtain a granular formulation.

(Formulation Example 4) Flowable formulation

| | |
|---|---|
| Substance (A) | 10 parts |
| Polyoxyethylene aryl phenyl ether | 2 parts |
| Dialkyl sulfosuccinate sodium salt | 0.5 part |
| Glycerin | 5 parts |
| Xanthan gum | 0.3 part |
| Water | 82.2 parts |

The above materials are mixed and wet ground to obtain a flowable formulation.

(Formulation Example 5) Water dispersible granule

| | |
|---|---|
| Substance (A) | 30 parts |
| Inorganic carrier | 70 parts |

The above materials are uniformly mixed, finely ground, and then granulated to obtain a water dispersible granule.

Test Example 1

Evaluation test for relief effects of high temperature injury on tomato

*N,N*-dimethylformamide based solutions were prepared according to the formulations shown in Table 3 to give chemical solutions for the tests.

Tomato (breed: Momotaro) grown up to the 2.5 leaf stage in a greenhouse was prepared.

The above chemical solution was sprayed to the stem and leaf parts of the above tomato nursery plants in a sufficient amount, and then air dried. They were allowed to grow at 30°C for 2 days under conditions of 16 hours under a daylight condition and 8 hours under a dark condition. Then, they were allowed to grow for 6 days under conditions of 16 hours under a daylight condition at 40°C and 8 hours under a dark condition at 30°C.

Subsequently, a degree of leaf browning and growth inhibition were observed to investigate states of high temperature injury.

Disorders were evaluated by 11 levels of disorder indices from 0 (no disorders) to 10 (withering to death).

High temperature injury relief percentages as compared with a region treated with solvent DMF only (Chemical solution 3) were computed by the following formula.

High temperature injury relief percentage = ((disorder index of region treated with solvent only) - (disorder index of each treatment region)) / (disorder index of region treated with solvent only) × 100
The results are shown in Table 3.

[Table 3]

Table 3

| | Chemical solution | | |
|---|---|---|---|
| | 1 | 2 | 3 |
| Substance(A)[Conc.ppm] ascorbyl palmitate | 800 | 0 | 0 |
| Agric.chemical[Conc.ppm] Pyraclostrobin | 0 | 40 | 0 |
| | | | |
| relief percents of high-temperature injury(%) | 57 | 35 | 0 |

Test Example 2

Evaluation test for relief effects of high temperature injury on cherry tomato

Cherry tomato (breed; Regina, 5 reprications) grown in a greenhouse was prepared.
On August 24 at the first inflorescence anthesis, 0.15% of 4-chlorophenoxyacetic acid was sprayed, and a water dispersible granule of 30% ascorbyl palmitate in an amount described in Table 4 was further applied at the plant foot at intervals of seven days. All fruits were harvested on September 28, and the color of a fruit (classified into red and green), the weight of a fruit, and the number of fruits were investigated to calculate the weight per fruit and the percentage of red fruits (%).
The results are shown in Table 4.
[Table 4]

Table 4

| | | Substance(A) | | no treatment |
|---|---|---|---|---|
| | | ascorbyl palmitate | | |
| dosage(g/stock) | | 0.5 | 0.1 | - |
| yield | Total weight of fruits(g) | 342 | 304 | 262 |
| | Total number of fruits | 33 | 32 | 38 |
| | weight per fruit(g/fruit) | 10.4 | 9.5 | 6.9 |
| | vs. no treatment region(%) | 151 | 138 | 100 |
| color of fruit | Number of red fruits | 29 | 19 | 16 |
| | Number of green fruits | 4 | 13 | 22 |
| | Percents of red fruits(%) | 88 | 59 | 42 |
| | vs. no treatment region(%) | 209 | 141 | 100 |

Test Example 3

Evaluation test for relief effects of low temperature injury on cucumber

N,N-dimethylformamide based solutions were prepared according to the formulations shown in Tables 5 to give chemical solutions for the tests.
Cucumber nursery plants (breed: Sagamihanjiro) grown up to the 1.5 leaf stage in a greenhouse was prepared.
The above chemical solution was sprayed to the stem and leaf parts of the above cucumber nursery plants in a sufficient amount, and then air dried. They were allowed to grow at 25°C for 2 days under conditions of 16 hours under a daylight condition and 8 hours under a dark condition. Then, they were allowed to grow for 9 days under conditions of 16 hours under a daylight condition at 10°C and 8 hours under a dark condition at 7°C.
Subsequently, a degree of leaf browning and outgrowth inhibition were observed to investigate states of low temperature injury.
Disorders were evaluated by 11 levels of disorder indices from 0 (no disorders) to 10 (withering to death).
Low temperature injury relief percentages as compared with a region treated with solvent DMF only (Chemical solution 3) were computed by the following formula.

Low temperature injury relief percentage = ((disorder index of region treated with solvent only) - (disorder index of each treated region)) / (disorder index of region treated with solvent only) × 100

The results are shown in Table 5.
[Table 5]

Table 5

| | Chemical solution | | |
|---|---|---|---|
| | 1 | 2 | 3 |
| Substance(A)[Conc.ppm] ascorbyl palmitate | 800 | 0 | 0 |
| Agric.chemical[Conc.ppm] Pyraclostrobin | 0 | 40 | 0 |
| | | | |
| relief percents of low-temperature injury(%) | 25 | 19 | 0 |

Test Example 4

Evaluation test for relief effects of low temperature injury on eggplant

Eggplant (breed: Senryo 2 gou, 3 reprications) grown up to the 4 to 6 leaf stage in a greenhouse was prepared.
A water dispersible granule of 30% ascorbyl palmitate and pyraclostrobin dissolved to 40% with N, N-dimethylformamide were diluted with tap water to a concentration described in Table 6, and the diluted solution was sprayed over the whole nursery plants in a sufficient amount. After air dried, they were allowed to grow for 1 day under conditions of 16 hours under a daylight condition at 18°C and 8 hours under a dark condition at 13°C, and then allowed to grow under conditions of 16 hours under a daylight condition at 13°C and 8 hours under a dark condition at 8°C. A degree of disorder was investigated at the elapsed time of 15 days after the spray treatment.
Disorders were evaluated by 4 levels of disorder indices of 0 (no color change), 1 (discolored up to 1/4 of the whole), 2 (discolored up to 1/2 of the whole), 3 (discolored at 1/2 or more of the whole) with reference to the area of a discolored portion in an expanded leaf after treatment.

Injury relief percentage = ((disorder index of untreated region) - (disorder index of each treated region)) / (disorder index of untreated region) × 100

The results are shown in Table 6.

[Table 6]

Table 6

| | Chemical solution | | |
|---|---|---|---|
| | 4 | 5 | 3 |
| Substance(A)[Conc.ppm] ascorbyl palmitate | 1000 | 0 | 0 |
| Agric.chemical[Conc.ppm] Pyraclostrobin | 0 | 50 | 0 |
| | | | |
| relief percents of low-temperature injury(%) | 13.0 | 13.0 | 0.0 |

Test Example 5

Evaluation test for relief effects of low temperature injury on tomato

Tomato (breed: Reiyo, 4 reprications) grown up to the 3.5 leaf stage in a greenhouse was prepared.

A water dispersible granule of 30% ascorbyl palmitate and pyraclostrobin dissolved to 40% with *N*,*N*-dimethylformamide were diluted with tap water to a concentration described in Table 7, and the diluted solution was sprayed over the whole nursery plants in a sufficient amount. After air dried, they were transferred to the outside of the greenhouse at an average temperature of 0.5°C during night to investigate a degree of low temperature injury on the next morning.

Disorders were evaluated by 5 levels of disorder indices of 0 (no disorder) to 4 (complete withering). From this, disorder relief percentages were computed by the following formula.

Low temperature injury relief percentage = (disorder index of untreated region) - (disorder index of each treatment region) / (disorder index of untreated region) × 100

The results are shown in Table 7.

[Table 7]

Table 7

| | Chemical solution | | |
|---|---|---|---|
| | 6 | 5 | 3 |
| Substance(A)[Conc.ppm] ascorbyl palmitate | 500 | 0 | 0 |
| Agric.chemical[Conc.ppm] Pyraclostrobin | 0 | 50 | 0 |
| | | | |
| relief percents of low-temperature injury(%) | 100 | 25 | 0 |

Test Example 6

Evaluation test for relief effects of high temperature injury on Eustoma grandiflorum

Eustoma grandiflorum (breed: King of Snow) grown at a temperature of 22°C and 16 hours under dark condition in a cell tray in the room was prepared. When an appropriately half were sprouted after seeding, a water dispersible granule of 30% ascorbyl palmitate was diluted with distilled water to a predetermined concentration, and the diluted solution was sprayed on the whole nursery plants in a sufficient amount. Subsequently, spraying by the same method was performed two times a week for the total of 10 times including the first application. Meanwhile, they were transferred to under conditions of 16 hours under a daylight condition at 35°C and 8 hours under a dark condition at 15°C at the expansion stage of a pair of true leaves 3 weeks after seeding, and allowed to grow for 2 weeks. Further, they were transferred to in a glass greenhouse and allowed to grow. Then naturalization was performed upon the expansion stage of two pairs of true leaves 8 week after seeding, and continuously allowed to grow in the greenhouse. Subsequently, the number of those bloomed out of the bolting plants was investigated.
The results are shown in Table 8.
[Table 8]

Table 8

| | Chemical solution | |
|---|---|---|
| | 7 | 8 |
| Substance(A)[Conc.ppm] ascorbyl palmitate | 600 | 0 |
| Number of bolting plants | 16 | 14 |
| Number of bloomed plants | 12 | 5 |
| Percents of bloomed plants(%) | 75.0 | 35.7 |

Test Example 7

Evaluation test for relief effects of strong light injury on tomato

Tomato (breed: Reiryo, 2 reprications) grown up to the two leaf stage in a greenhouse was prepared.
A water dispersible granule of 30% of ascorbyl palmitate and pyraclostrobin dissolved to 40% N,N-dimethylformamide were diluted with tap water to each of the concentrations described in Table 9, and the diluted solution was sprayed over the whole nursery plants in a sufficient amount. After air dried, they were exposed to strong light under summer blazing sun. A degree of disorder was investigated at the elapsed time of 4 days after the spraying.
For disorder, a degree of necrosis due to light effects was evaluated by 11 levels of disorder indices from 0 (no necrosis) to 10 (withering to death). From this, disorder relief percentages were computed by the following formula.

Injury relief percentage = ((disorder index of untreated region) - (disorder index of each treated region)) / (disorder index of untreated region) × 100

The results are shown in Table 9.
[Table 9]

Table 9

| | Chemical solution | | |
|---|---|---|---|
| | 4 | 5 | 3 |
| Substance(A)[Conc.ppm] ascorbyl palmitate | 1000 | 0 | 0 |
| Agric.chemical[Conc.ppm] Pyraclostrobin | 0 | 50 | 0 |

(continued)

| | Chemical solution | | |
|---|---|---|---|
| | 4 | 5 | 3 |
| | | | |
| relief percents of strong light injury(%) | 50 | 40 | 0 |

Test Example 8

Evaluation test for relief effects of phytotoxicity on tomato

N,N-dimethylformamide based solutions were prepared according to the formulations shown in Table 10 to give chemical solutions for the tests.

Tomato nursery plants (breed: Momotaro) grown up to the 4 leaf stage in a greenhouse were prepared.

The above chemical solution was sprayed to the stem and leaf parts of the above tomato nursery plants in an amount of liquid dropping, and then air dried. They were allowed to grow for 7 days under the average temperature and humidity conditions on March in Japan.

Subsequently, phytotoxicity such as a degree of leaf browning and outgrowth inhibition was investigated.

Phytotoxicity was evaluated by 11 levels of phytotoxicity indices of 0 (with no disorder) to 10 (withering to death).

Chemical poisoning injury relief percentages as compared with a region treated with solvent DMF only were computed by the following formula.

Chemical poisoning injury relief percentage = (phytotoxicity index of region treated with solvent only) - (phytotoxicity index of each treated region)) / (phytotoxicity index of region treated with solvent only) × 100

The results are shown in Table 10.

[Table 10]

Table 10

| | Chemical solution | | | | | | | |
|---|---|---|---|---|---|---|---|---|
| | 9 | 10 | 11 | 12 | 13 | 14 | 15 | 16 |
| Substance(A)[Conc.ppm]<br>ascorbyl palmitate | 800 | 800 | 0 | 0 | 800 | 800 | 0 | 0 |
| Agric.chemical[Conc.ppm]<br>Fluazinam<br>Azoxystrobin | <br>200<br>0 | <br>100<br>0 | <br>200<br>0 | <br>100<br>0 | <br>0<br>200 | <br>0<br>100 | <br>0<br>200 | <br>0<br>100 |
| | | | | | | | | |
| phytotoxicity index | 4 | 2 | 6 | 6 | 4 | 3 | 6 | 5 |
| relief percents of phytotoxicity (%) | 33 | 67 | 0 | 0 | 33 | 40 | 0 | 0 |

Test Example 9

Test for green color maintenance effects (relief of high temperature injury) on wheat

Wheat (breed, Norin No. 61, 15 plants/$m^2$/region, 2 reprications) grown in the field was used. A water dispersible

granule of 30% ascorbyl palmitate in an amount described in Table 11 was applied to the plant foot 4 times at intervals of seven days from the next day of the ear emergence day in August. The leaf color index of top 4 to 5 leaves of each plant in a treatment region was investigated on September 6 to evaluate maintenance effects of leaf color as compared with that in the untreated region.

Leaf color was evaluated by 4 levels of leaf color indices of 1 (discolored at 1/4 or less of the whole), 2 (discolored at 1/2 or less of the whole) and 3 (discolored at 3/4 or more of the whole). From this, the mean leaf color index, and green color maintenance effects were computed by the following formula.

$$\text{Green color maintenance effect} = \text{(leaf color index of untreated region) - (leaf color index of each treatment region)) / (leaf color index of untreated region)} \times 100$$

The results are shown in Table 11.

[Table 11]

Table 11

| | Substance(A) ascorbyl palmitate | no treatment |
|---|---|---|
| appl. amount (g/m$^2$) | 1.5 | - |
| mean leaf color index | 1.5 | 2.0 |
| Green color maintenance effect(%) | 25.0 | 0.0 |

Test Example 10

Evaluation test for relief effects of submergence disorder on cucumber

Cucumber (breed: Sagamihanjirohushinari, 2 reprications) grown up to the two leaf stage in a greenhouse was prepared.

A water dispersible granule of 30% ascorbyl palmitate and pyraclostrobin adjusted to 40% with N, N-dimethylformamide were diluted with tap water to a predetermined concentration, and the diluted solution was sprayed in a sufficient amount. They were subjected to flood conditions up to immediately below the cotyledon from 2 days after the spray treatment, and the raw weights of an above ground part and a root part of cucumber were each measured at the elapsed time of 11 days after the spray treatment. From this, injury relief percentages were computed by the following formula.

$$\text{Flood injury relief percentage} = \text{((raw weight of each treatment region) - (raw weight of untreated region)) / (raw weight of untreated region)} \times 100$$

The results are shown in Table 12.

[Table 12]

Table 12

| | Chemical solution | | |
|---|---|---|---|
| | 4 | 5 | 3 |
| Substance(A)[Conc.ppm] ascorbyl palmitate | 1000 | 0 | 0 |

(continued)

| | Chemical solution | | |
|---|---|---|---|
| | 4 | 5 | 3 |
| Agric.Chemical[Conc.ppm] Pyraclostrobin | 0 | 50 | 0 |
| | | | |
| relief percents of flood injury in aerial part(%) | 31.9 | 61.7 | 0.0 |
| relief percents of flood injury in root(%) | 41.5 | 39.0 | 0.0 |

Test Example 11

Evaluation test for relief effects of flood injury on soybean

Soybean (breed: Enrei, 2 reprications) grown up to the two leaf stage in a greenhouse was prepared.

A water dispersible granule of 30% ascorbyl palmitate and pyraclostrobin adjusted to 40% with *N,N*-dimethylformamide were diluted with tap water to a predetermined concentration, and the diluted solution was sprayed in a sufficient amount. They were subjected to flood conditions up to immediately below the cotyledon from 2 days after the spray treatment, and the raw weights of an above ground part and a root part of soybean were each measured at the elapsed time of 11 days after the spray treatment. From this, injury relief percentages were computed by the following formula.

$$\text{Flood injury relief percentage} = ((\text{raw weight of each treatment region}) - (\text{raw weight of untreated region})) / (\text{raw weight of untreated region}) \times 100$$

The results are shown in Table 13.

[Table 13]

Table 13

| | Chemical solution | | |
|---|---|---|---|
| | 4 | 5 | 3 |
| Substance(A)[Conc.ppm] ascorbyl palmitate | 1000 | 0 | 0 |
| Agric.Chemical[Conc.ppm] Pyraclostrobin | 0 | 50 | 0 |
| | | | |
| relief percents of flood injury in aerial part(%) | 2.8 | 0.0 | 0.0 |
| relief percents of flood injury in root(%) | 20.4 | 3.2 | 0.0 |

Test Example 12

Evaluation test for relief effects of acidity problem on cucumber

Cucumber (breed: Sagamihanjirohushinari, 2 reprications) hydroponically grown up to the two leaf stage in a 100 ml flask was prepared.

A water dispersible granule of 30% ascorbic acid palmitate and pyraclostrobin adjusted to 40% with N, N-dimethylformamide were diluted with tap water to a predetermined concentration, and the diluted solution was sprayed over the whole nursery plants in a sufficient amount. The water culture medium was adjusted to pH 4 with 1 N hydrochloric acid at the elapsed time of 2 days after the spray treatment, and the above cucumber was continuously allowed to grow hydroponically. Leaf stage of the cucumber was investigated 17 days after the spray treatment. From this, disorder relief

percentages were computed by the following formula.

Acidity problem relief percentage = ((leaf stage of each treatment region) - (leaf stage of untreated region)) / (leaf stage of untreated region) × 100

The results are shown in Table 14.
[Table 14]

Table 14

| | Chemical solution | | |
|---|---|---|---|
| | 4 | 5 | 3 |
| Substance(A)[Conc.ppm] ascorbyl palmitate | 1000 | 0 | 0 |
| Agric.Chemical[Conc.ppm] Pyraclostrobin | 0 | 50 | 0 |
| | | | |
| relief percents of acidity problem(%) | 15.6 | 15.6 | 0.0 |

Test Example 13

Evaluation test for relief effects of acidity problem on soybean

Soybean (breed: Enrei, 2 reprications) hydroponically grown up to the two leaf stage in a 100 ml flask was prepared.
A water dispersible granule of 30% ascorbyl palmitate and pyraclostrobin dissolved to 40% with N, N-dimethylformamide were diluted with tap water to a predetermined concentration, and the diluted solution was sprayed over the whole nursery plants in a sufficient amount. The water culture medium was adjusted to pH 4 with 1 N hydrochloric acid at the elapsed time of 2 days after the spray treatment, and the above soybean was continuously allowed to grow hydroponically. Disorder in the soybean was investigated at the elapsed time of 11 days after the spray treatment.
For disorder, a degree of necrosis was evaluated by 11 levels of disorder indices from 0 (no necrosis) to 10 (withering to death). From this, problem relief percentages were computed by the following formula.

Acidity problem relief percentage = ((disorder index of untreated region) - (disorder index of each treated region)) / (disorder index of untreated region) × 100

The results are shown in Table 15.
[Table 15]

Table 15

| | Chemical solution | | |
|---|---|---|---|
| | 4 | 5 | 3 |
| Substance(A)[Conc.ppm] ascorbyl palmitate | 1000 | 0 | 0 |
| Agric.Chemical[Conc.ppm] Pyraclostrobin | 0 | 50 | 0 |

(continued)

| | Chemical solution | | |
|---|---|---|---|
| | 4 | 5 | 3 |
| | | | |
| relief percents of acidity problem(%) | 43.8 | 12.5 | 0.0 |

Test Example 14

Evaluation test for relief effects of salt injury on cucumber

Cucumber (breed: Sagamihanjirohushinari, 2 reprications) hydroponically grown up to the two leaf stage in a greenhouse was prepared.

A water dispersible granule of 30% ascorbic acid palmitate and pyraclostrobin adjusted to 40% with N, N-dimethylformamide were diluted with tap water to a predetermined concentration, and the diluted solution was sprayed over the whole nursery plants in a sufficient amount. After air dried, they were cultivated with normal irrigation in a greenhouse. Then, the irrigation conditions were changed to 0.1% aqueous sodium chloride solution in 2 cm depth from 2 days after the spraying, and cultivated up to 11 days after the spray treatment. Then the raw weights of an aerial part and a root part were each measured. From this, injury relief percentages were computed by the following formula.

Salt injury relief percentage = ((raw weight of each treatment region) - (raw weight of untreated region)) / (raw weight of untreated region) × 100

The results are shown in Table 16.

[Table 16]

Table 16

| | Chemical solution | | |
|---|---|---|---|
| | 4 | 5 | 3 |
| Substance(A)[Conc.ppm] ascorbyl palmitate | 1000 | 0 | 0 |
| Agric.Chemical[Conc.ppm] Pyraclostrobin | 0 | 50 | 0 |
| | | | |
| relief percents of salt injury in aerial part(%) | 8.8 | 45.6 | 0.0 |
| relief percents of salt injury in root(%) | 16.0 | 20.0 | 0.0 |

Test Example 15

Evaluation test for relief effects of salt injury on soybean

Soybean (breed: Enrei, 2 reprications) hydroponically grown up to the two leaf stage in a greenhouse was prepared.

A water dispersible granule of 30% ascorbic acid palmitate and pyraclostrobin adjusted to 40% with N, N-dimethylformamide were diluted with tap water to a predetermined concentration, and the diluted solution was sprayed over the whole nursery plants in a sufficient amount. After air dried, they were cultivated with normal irrigation in a greenhouse. Then, the irrigation conditions were changed to 0.1% aqueous sodium chloride solution in 2 cm depth from 2 days after the spraying, and cultivated up to 11 days after the spray treatment. Then the raw weights of an aerial part and a root part were each measured. From this, injury relief percentages were computed by the following formula.

$$\text{Salt injury relief percentage} = ((\text{raw weight of each treatment region}) - (\text{raw weight of untreated region})) / (\text{raw weight of untreated region}) \times 100$$

The results are shown in Table 17.

[Table 17]

Table 17

| | Chemical solution | | |
|---|---|---|---|
| | 4 | 5 | 3 |
| Substance(A)[Conc.ppm] ascorbyl palmitate | 1000 | 0 | 0 |
| Agric.Chemical[Conc.ppm] Pyraclostrobin | 0 | 50 | 0 |
| | | | |
| relief percents of salt injury in aerial part(%) | 20.4 | 3.2 | 0.0 |
| relief percents of salt injury in root(%) | 22.2 | 2.5 | 0.0 |

Test Example 16

Test for symptom relief effects against tomato yellow leaf curl virus disease

N,N-dimethylformamide based solutions were prepared according to the formulations shown in Table 18 to give chemical liquids for the tests.

Tomato nursery plants (breed: Momotaro) grown up to the 8 leaf stage in a greenhouse were prepared.

Tomato nursery plants suffering from tomato yellow leaf curl virus (TYLCV) were used as inoculation sources.

A diseased plant was obliquely cut in round slices at the stem, and graft-inoculated to a tomato nursery plant. In order to prevent dryness, parafilm was wrapped around the grafted portion for protection.

After the graft inoculation, the above chemical solution was sprayed on the stem and leaf part of the tomato nursery plant in a sufficient amount. Then, the above chemical solution was sprayed 3 times at intervals of about one week in a sufficient amount. Symptoms of tomato yellow leaf curl disease were investigated at an elapsed time of 25 days.

Symptoms were evaluated by 5 levels of onset indices of 0 (with no onset) to 4 (fulminant).

Onset inhibition percentages as compared with a region treated with solvent DMF only (Chemical solution 3) were computed by the following formula.

$$\text{Onset inhibition percentage} = (\text{onset index of region treated with solvent only}) - (\text{onset index of each treatment region})) / (\text{onset index of region treated with solvent only}) \times 100$$

Further, the expectation values of onset inhibition percentages were computed based on the Colby's equation.

The Colby's equation is $E = M + N - MN/100$. In the equation, E represents the expectation value of an onset inhibition percentage (%), and M represents an onset inhibition percentage (%) calculated from measurements when the substance (A1) is used alone, and N represent an onset inhibition percentage (%) calculated from measurements when the substance (A2) is used alone. The results are shown in Table 18.

[Table 18]

Table 18

| | Chemical solution | | | | |
|---|---|---|---|---|---|
| | 17 | 4 | 6 | 18 | 3 |
| Substance(A1)[Conc.ppm] Ascorbyl glucoside | 500 | 0 | 0 | 500 | 0 |
| Substance(A2)[Conc.ppm] ascorbyl palmitate | 500 | 1000 | 500 | 0 | 0 |
| | | | | | |
| Onset percentage(%) | 10 | 20 | 30 | 40 | 50 |
| Onset inhibition percentage(%) | 80 | 60 | 40 | 20 | 0 |
| Expectation value(%) | 52 | 60 | 40 | 20 | 0 |

Test Example 17

Tests for relief effects of disease stress on rice plant

Nursery plants of rice (breed: Koshihikari, 10 reprications) were prepared. A water dispersible granule of 30% ascorbyl palmitate and pyraclostrobin adjusted to 5% with N,N-dimethylformamide were diluted with tap water to a predetermined concentration, and these were sprayed over the whole nursery plants in a sufficient amount, and then air dried. They were inoculated with Pyricularia oryzae on the next day. The number of rice blast spots was investigated at the elapsed time of 11 days after the inoculation. From this, preventive values were computed by the following formula.

$$\text{Preventive value} = ((\text{number of lesion spots in untreated region}) - (\text{number of lesion spots in each treated region})) / (\text{number of lesion spots in untreated region}) \times 100$$

The results are shown in Table 19.
[Table 19]

Table 19

| | Chemical solution | | |
|---|---|---|---|
| | 19 | 20 | 3 |
| Substance(A)[Conc.ppm] ascorbyl palmitate | 50 | 0 | 0 |
| Agric.Chemical[Conc.ppm] Pyraclostrobin | 0 | 5 | 0 |
| | | | |
| Number of lesion spots | 30 | 10 | 33 |
| Preventive value(%) | 9 | 70 | 0 |

## Claims

1. A method of providing a plant with stress resistance, wherein the method comprises
applying at least one substance (A) to the plant, said at least one substance (A) being selected from the group consisting of
compounds represented by Formula (I):

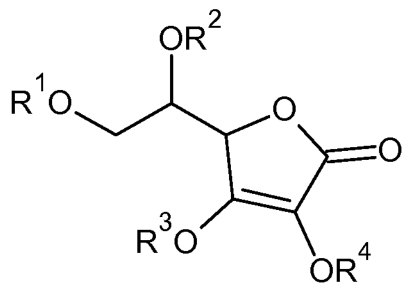

[In Formula (I), $R^1$ to $R^4$ each independently represents a hydrogen atom, $-SO_3H$, $-PO_3H_2$, a glycosyl group or $-COR^{11}$. $R^{11}$ represents an unsubstituted or substituted C1 to C30 alkyl group or an unsubstituted or substituted C2 to C30 alkenyl group.],
compounds represented by Formula (II):

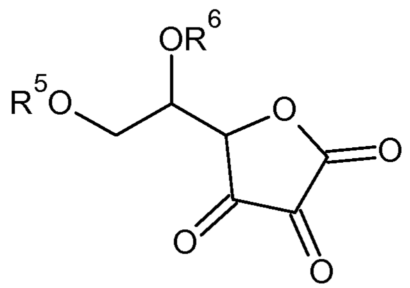

[In Formula (II), $R^5$ and $R^6$ each independently represents a hydrogen atom, $-SO_3H$, $-PO_3H_2$, a glycosyl group or $-COR^{11}$. $R^{11}$ represents an unsubstituted or substituted C1 to C30 alkyl group or an unsubstituted or substituted C2 to C30 alkenyl group.], and salts thereof.

2. The method according to claim 1, wherein the substance (A) is a compound represented by Formula (I) [provided that $R^1$ to $R^4$ are not each simultaneously a hydrogen atom.] or a salt thereof.

3. The method according to claim 1, wherein the substance (A) is a compound represented by Formula (I) [provided that at least one of $R^1$ to $R^4$ represents $-COR^{11}$, and $R^{11}$ represents an unsubstituted or substituted C12 to C30 alkyl group or an unsubstituted or substituted C12 to C30 alkenyl group.] or a salt thereof.

4. The method according to claim 1, wherein the substance (A) is a compound represented by Formula (I) [provided that $R^1$ to $R^4$ each independently represents a hydrogen atom or $-COR^{11}$, and at least one of $R^1$ to $R^4$ represents $-COR^{11}$. $R^{11}$ represents an unsubstituted or substituted C1 to C30 alkyl group or an unsubstituted or substituted C2 to C30 alkenyl group. $R^{11}$ in at least one of $-COR^{11}$ represents an unsubstituted or substituted C12 to C30 alkyl group or an unsubstituted or substituted C12 to C30 alkenyl group.] or a salt thereof.

5. The method according to claim 1, wherein the substance (A) is a composition comprising a water soluble substance (A1) of those selected from the group consisting of compounds represented by Formula (I), compounds represented by Formula (II) and salts thereof; and a lipid soluble substance (A2) of those selected from the group consisting of compounds represented by Formula (I), compounds represented by Formula (II) and salts thereof.

6. The method according to any one of claims 1 to 5, wherein the stress is at least one of biological stress due to plant viruses, phytopathogenic bacteria, phytopathogenic filamentous fungi, agricultural pests or weeds; or physical or chemical stress due to high temperature, low temperature, high illuminance, low illuminance, excessive humidity, dryness, salt, acidity, agricultural chemicals, chemical substances or heavy metals.

7. A stress resistance conferring composition for a plant, wherein the composition comprises at least two substances (A) selected from the group consisting of
compounds represented by Formula (I):

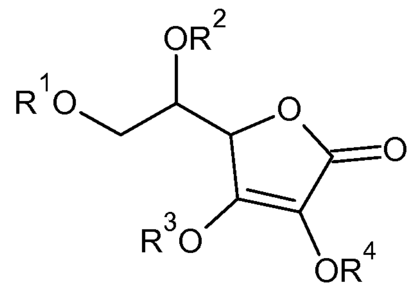

[In Formula (I), $R^1$ to $R^4$ each independently represents a hydrogen atom, $-SO_3H$, $-PO_3H_2$, a glycosyl group or $-COR^{11}$. $R^{11}$ represents an unsubstituted or substituted C1 to C30 alkyl group or an unsubstituted or substituted C2 to C30 alkenyl group.],
compounds represented by Formula (II):

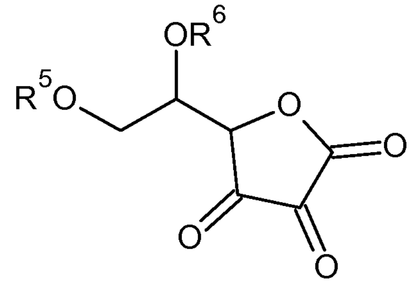

[In Formula (II), $R^5$ and $R^6$ each independently represents a hydrogen atom, $-SO_3H$, $-PO_3H_2$, a glycosyl group or $-COR^{11}$. $R^{11}$ represents an unsubstituted or substituted C1 to C30 alkyl group or an unsubstituted or substituted C2 to C30 alkenyl group.], and
salts thereof.

**8.** The composition according to claim 7, wherein one of the substances (A) is a water soluble substance (A1) of those selected from the group consisting of compounds represented by Formula (I), compounds represented by Formula (II) and salts thereof; and another of the substances (A) is a lipid soluble substance (A2) of those selected from the group consisting of compounds represented by Formula (I), compounds represented by Formula (II) and salts thereof.

**9.** A stress resistance conferring composition for a plant, wherein the composition comprises at least one water soluble substance (A1) selected from the group consisting of
compounds represented by Formula (Ia):

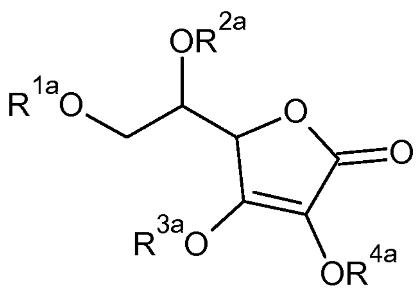

[In Formula (Ia), $R^{1a}$ to $R^{4a}$ each independently represents a hydrogen atom, $-SO_3H$, $-PO_3H_2$ or a glycosyl group.],
compounds represented by Formula (IIa):

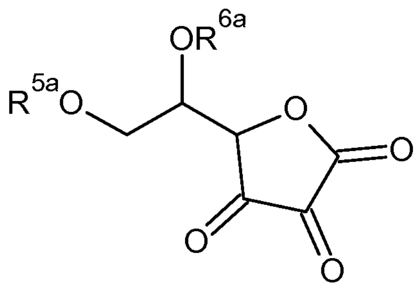

[In Formula (IIa), $R^{5a}$ and $R^{6a}$ each independently represents a hydrogen atom, $-SO_3H$, $-PO_3H_2$ or a glycosyl group.], and salts thereof; and
at least one lipid soluble substance (A2) selected from the group consisting of
compounds represented by Formula (Ib):

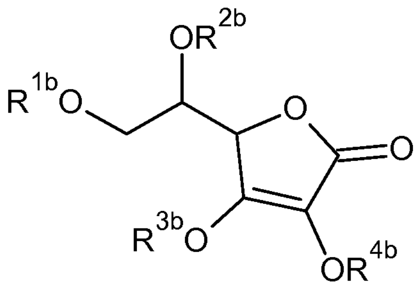

[In Formula (Ib), $R^{1b}$ to $R^{4b}$ each independently represents a hydrogen atom or $-COR^{11}$. At least one of $R^{1b}$ to $R^{4b}$ represents $-COR^{11}$, and $R^{11}$ represents an unsubstituted or substituted C1 to C30 alkyl group or an unsubstituted or substituted C2 to C30 alkenyl group.],
compounds represented by Formula (IIb):

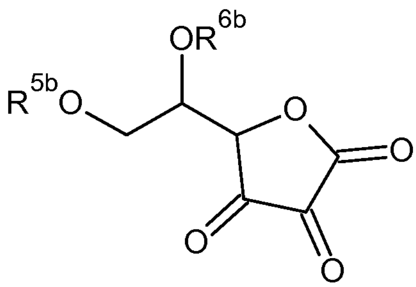

[In Formula (IIb), $R^{5b}$ and $R^{6b}$ each independently represents a hydrogen atom or $-COR^{11}$. At least one of $R^{5b}$ and $R^{6b}$ represents $-COR^{11}$, and $R^{11}$ represents an unsubstituted or substituted C1 to C30 alkyl group or an unsubstituted or substituted C2 to C30 alkenyl group.], and salts thereof.

**10.** A method of reducing phytotoxicity of a plant due to an agricultural chemical, wherein the method comprises providing the plant with stress resistance by the method according to any one of claims 1 to 6.

**11.** The method of reducing phytotoxicity of a plant due to an agricultural chemical according to claim 10, wherein the agricultural chemical comprises at least one selected from the group consisting of fungicides, insecticides, plant growth regulators and herbicides.

## INTERNATIONAL SEARCH REPORT

International application No.
PCT/JP2013/004430

A. CLASSIFICATION OF SUBJECT MATTER
*A01N43/08*(2006.01)i, *A01G7/06*(2006.01)i, *A01N25/32*(2006.01)i, *A01N43/16*(2006.01)i, *A01N43/40*(2006.01)i, *A01N43/54*(2006.01)i, *A01P21/00*(2006.01)i

According to International Patent Classification (IPC) or to both national classification and IPC

B. FIELDS SEARCHED

Minimum documentation searched (classification system followed by classification symbols)
A01N43/08, A01G7/06, A01N25/32, A01N43/16, A01N43/40, A01N43/54, A01P21/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CAplus(STN), REGISTRY(STN), JSTPlus(JDreamIII), JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| A | JP 2008-538566 A (Goldstein, Glenn A.), 30 October 2008 (30.10.2008), claims; example 2 & WO 2006/132712 A2 & EP 1881761 A2 & US 2008/274888 A1 & CN 101203133 A | 1-11 |
| A | WO 2011/030816 A1 (National University Corporation Hokkaido University), 17 March 2011 (17.03.2011), claims & EP 2478768 A1 & US 2012/172580 A1 & CN 102480950 A | 1-11 |
| A | JP 04-342507 A (Meiji Seika Kaisha, Ltd.), 30 November 1992 (30.11.1992), claims (Family: none) | 1-11 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 August, 2013 (28.08.13) | 17 September, 2013 (17.09.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011030816 A
- JP 2001508808 A


### Non-patent literature cited in the description


- *Vitamins,* 2005, vol. 79 (2), 116-117
- *The Horticulture Journal,* vol. 6 (2), 169-175